(19) Europäisches Patentamt
European Patent Office
Office européen des brevets

(11) EP 2 654 802 B1

(12) **FASCICULE DE BREVET EUROPEEN**

(45) Date de publication et mention de la délivrance du brevet:
**06.12.2017 Bulletin 2017/49**

(21) Numéro de dépôt: **11802928.9**

(22) Date de dépôt: **20.12.2011**

(51) Int Cl.:
***A61K 49/18*** *(2006.01)*

(86) Numéro de dépôt international:
**PCT/EP2011/073448**

(87) Numéro de publication internationale:
**WO 2012/084981 (28.06.2012 Gazette 2012/26)**

(54) **NANOEMULSION DE CHELATE POUR IRM**

CHELATNANOEMULSION FÜR MRI

CHELATE NANOEMULSION FOR MRI

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.12.2010 FR 1060847**

(43) Date de publication de la demande:
**30.10.2013 Bulletin 2013/44**

(73) Titulaires:
- **Guerbet**
  **93420 Villepinte (FR)**
- **Université de Bordeaux 1**
  **33400 Talence (FR)**

(72) Inventeurs:
- **PORT, Marc**
  **F-95170 Deuil La Barre (FR)**
- **ROBIC, Caroline**
  **F-94130 Nogent Sur Marne (FR)**
- **LEAL CALDERON, Fernando**
  **F-33650 La Brede (FR)**
- **CHADEL, Samy**
  **F-69740 Genas (FR)**

(74) Mandataire: **Regimbeau**
**20, rue de Chazelles**
**75847 Paris Cedex 17 (FR)**

(56) Documents cités:
**WO-A1-95/33494 WO-A2-2006/100305**
**US-A1- 2007 148 194**

- **MIYAMOTO M ET AL: "PREPARATION OF GADOLINIUM-CONTAINING EMULSIONS STABILIZED WITH PHOSPHATIDYLCHOLINE-SURFACTANT MIXTURES FOR NEUTRON-CAPTURE THERAPY", CHEMICAL AND PHARMACEUTICAL BULLETIN, PHARMACEUTICAL SOCIETY OF JAPAN, TOKYO, JP, vol. 47, no. 2, 1 février 1999 (1999-02-01), pages 203-208, XP000804240, ISSN: 0009-2363**
- **KRISTOF KIMPE ET AL: "Potential MRI Contrast Agents Based on Micellar Incorporation of Amphiphilic Bis(alkylamide) Derivatives of [(Gd-DTPA)(H2O)]2-", EUROPEAN JOURNAL OF INORGANIC CHEMISTRY, vol. 2003, no. 16, 1 août 2003 (2003-08-01), pages 3021-3027, XP055002051, ISSN: 1434-1948, DOI: 10.1002/ejic.200300117**

EP 2 654 802 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

L'invention concerne de nouveaux systèmes optimisés de type nanoémulsions et leur utilisation comme agents de contraste notamment en IRM

Dans le domaine de l'imagerie diagnostique, un grand nombre de recherches ont concerné des nanosystèmes lipidiques de type émulsion. Typiquement les émulsions utilisées sont sous forme de vésicules préparées à l'aide de constituants lipidiques (huile en particulier) et de tensioactifs (également désignés surfactants) servant d'interface entre la phase aqueuse et le noyau lipidique de la nanoparticule. Les émulsions lipidiques huile dans eau incorporent une phase huileuse formant des gouttelettes lipidiques dispersées en solution aqueuse.

Une première catégorie d'émulsions décrite notamment dans WO 03/062198 ou US 6 676 963 est celle de nanoémulsions fluorées, comprenant, intégrées à l'intérieur des vésicules lipidiques, des composés fluorés. Le coeur lipidique est formé d'une huile fluorée, et entouré d'une couche de tensioactif (surfactant, lécithine par exemple). Ces émulsions fluorées peuvent comprendre en outre un nombre très élevé de complexes de métaux paramagnétiques, en particulier de lanthanides. On connaît ainsi des émulsions fluorées pour IRM incorporant des chélates capables de complexer des lanthanides en particulier le gadolinium. Les chélates utilisés sont notamment des dérivés du DTPA, DOTA, DO3A, HPDO3A et autres chélates largement décrits dans l'art antérieur. Ces chélates hydrophiles sont rendus amphiphiles en leur greffant une zone lipophile telle qu'un phospholipide, ce qui permet de les intégrer dans la couche lipidique que forme le tensioactif de la composition. Plusieurs milliers (5000 à 100 000 environ) de ces complexes sont intégrés dans la membrane lipidique de ces vésicules, ce qui permet d'obtenir une relaxivité (signal IRM) élevée pour une détection de la zone physiologique étudiée. La partie hydrophile (la partie hydrophile que représente le chélate auquel est attaché un groupe lipophile de manière à rendre le chélate amphiphile) est localisée à la surface externe des nanogouttelettes, en contact avec la phase aqueuse de la solution de nanogouttelettes.

En outre, afin d'obtenir un signal spécifique de zones pathologiques, par exemple associées à une surexpression d'un marqueur de ces zones (récepteurs par exemple), des molécules de ciblage (ou biovecteurs, peptide par exemple ayant une affinité pour le récepteur) ont été greffées sur les nanogouttelettes de ces émulsions fluorées.

Toutefois, malgré des avancées prometteuses, ces agents de contraste fluorés et vectorisés décrits n'ont pas encore démontré totalement leur efficacité clinique, et nécessitent un savoir-faire de fabrication à l'échelle industrielle bien spécifique pour l'utilisation de composés fluorés, en particulier pour l'incorporation des biovecteurs.

Une deuxième catégorie d'émulsions est celle des nanoémulsions pour l'imagerie de fluorescence, ne comprenant typiquement pas de composés fluorés, et utilisant des nanocristaux d'oxydes métalliques. Le document WO 2010/018222 décrit de telles nanoémulsions comprenant :

- une solution aqueuse
- une phase lipidique dispersée (huile) formant des nanogouttelettes lipidiques dans la solution aqueuse, les nanogouttelettes incorporant des nanocristaux typiquement d'oxydes métalliques ayant
- un surfactant (phospholipides par exemple) pour stabiliser les nanogouttelettes.

Les huiles suggérées par le WO 2010/018222 (désignées lipides de solubilisation dans ce document) sont des huiles saturées ou des huiles insaturées (soja, lin, palme, tournesol...). Une huile saturée préférée présentée en détail est la Suppocire® (Gattefosse), huile saturée qui comprend une quantité très faible de glycérides en C8-C10 (moins de 2%). Cette huile solide à température ambiante, fluide à température corporelle, est autorisée chez l'homme, mais ne peut être utilisée pour former une composition de produit de contraste injectable à administration intra veineuse (qui doit être fluide à température ambiante). La teneur en phase lipidique dispersée de ces émulsions est très variable, indiquée comme comprise entre 20 et 40%. Les exemples indiquent une quantité élevée de surfactants (de l'ordre de 20% en poids de composition). Il est fait mention de l'ajout possible, en plus des nanocristaux et non à leur place, de chélates de lanthanides. Ces émulsions comprennent nécessairement en outre un co-surfactant (myrj® notamment) destiné à améliorer le contrôle de la taille et de la stabilité physico-chimique dans le temps (au moins 6 mois) des nanoémulsions. En effet sans ce co-surfactant, les propriétés ne sont pas satisfaisantes comme l'expliquent en détail les auteurs de ce document.

Une troisième catégorie d'émulsions est celle des nanoémulsions essentiellement thérapeutiques (encapsulation de médicaments), sans coeur fluoré, et dont certaines variantes sont décrites comme utilisables pour de l'imagerie IRM. Le document US 2007/0148194 décrit de telles émulsions pouvant incorporer des chélates de lanthanides en particulier de gadolinium. Ces émulsions huile/eau comprennent :

- une solution aqueuse
- une phase lipidique dispersée (huile) formant des nanogouttelettes lipidiques dans la solution aqueuse
- des surfactants (phospholipides par exemple) pour stabiliser les nanogouttelettes.

Ce document décrit spécifiquement l'utilisation d'huile riche en acides oméga 3 et 6 notamment (acide linoléique en particulier). Les huiles utilisées sont des huiles insaturées riches en acides gras à longue chaîne (acides en C18). Le taux d'acides gras à courtes chaînes (C8 et C10 notamment) est très faible. La teneur en phase lipidique dispersée de ces émulsions est très variable, indiquée comme comprise entre 5 et 40%, et la gamme possible de surfactants est très large (0.5 à 15% en poids de la composition).

Ce document ainsi que sa procédure d'examen accessible au public insistent sur l'importance d'utiliser ces huiles insaturées ayant une concentration d'au moins 20% en acides gras polyinsaturés oméga 3, pour obtenir l'effet biologique recherché, et plus exactement le passage des barrières biologiques des organes sans effet toxique pour les organes ou les tissus.

Toutefois, ces compositions utilisant des huiles polyinsaturées posent plusieurs problèmes techniques :

- les huiles polyinsaturées oméga 3 et/ou oméga 6 ne sont pas bien adaptées pour des formulations pharmaceutiques injectables d'agents de contraste
- les huiles insaturées sont sensibles à l'oxydation, d'où d'une part un problème de stabilité de l'émulsion dans la durée, notamment pour une conservation de plusieurs mois (typiquement pendant 3 ans pour les agents de contraste injectables), et d'autre part un risque (lié à la présence d'oxygène) d'altération du comportement paramagnétique du produit pour les examens d'imagerie médicale IRM

De plus, une quantité de surfactant d'au moins environ 3% de la composition en poids, et notamment de l'ordre de 3% à 5% se traduit par :

- la formation dans la composition, en plus des nanogouttelettes, de micelles (nanoparticules dépourvues de coeur huileux) dont le retrait nécessiterait pour une production à l'échelle industrielle de centaines de tonnes de produit de contraste, des étapes de séparation et de purification complexes et coûteuses et donc une chute du rendement industriel
- un risque d'une quantité trop élevée de chélates de lanthanides administrée au patient, avec les risques de tolérance dus aux lanthanides libres en solution que veut absolument éviter l'homme du métier
- la difficulté voire l'impossibilité d'incorporer aux nanoparticules une quantité appropriée de chélates et de biovecteurs de ciblage biologique dont le coût est très élevé : les tensioactifs lipides amphiphiles ont un pouvoir tensioactif plus fort que les biovecteurs amphiphiles et vont préférentiellement former la couche autour de l'huile (et/ou la couche de lipides amphiphiles surfactants se forme à partir de ces lipides avant même que les biovecteurs n'aient le temps de s'intégrer au sein de cette couche).

Encore plus précisément :

- lorsque la quantité totale de surfactants (surfactant type lipoïde ou analogue, chélate amphiphile, biovecteur amphiphile) formant des nanogouttelettes est atteinte, les composés amphiphiles de la solution viennent former rapidement des micelles, et la solution contient alors beaucoup plus de micelles que de nanogouttelettes
- le prix de revient industriel des nanoémulsions est pour environ au moins 80 à 90% représenté par le biovecteur rendu amphiphile, on comprend donc qu'une perte de biovecteurs génère des surcoûts industriels beaucoup trop importants
- si la quantité de lipides amphiphiles tensioactifs (composés non vectorisés) est trop importante, les biovecteurs amphiphiles ne peuvent s'intégrer de manière satisfaisante dans la couche amphiphile autour de l'huile, ce qui génère une perte d'affinité très importante et rend le produit inapproprié pour un ciblage spécifique du territoire pathologique.

D'ailleurs, ce document US 2007/0148194 décrit l'utilisation de molécules pharmaceutiques utilisés comme médicament de traitement thérapeutique, et non comme agent de vectorisation de la nanoémulsion pour une imagerie moléculaire spécifique. Ce document antérieur distingue d'une part le médicament thérapeutique (paclitaxel par exemple), et l'agent de contraste (chélate de lanthanide). Selon la compréhension du demandeur de ce document au vu de l'état de la technique, de telles nanoémulsions n'atteignent pas le territoire biologique cible à l'aide de biovecteurs de ciblage. Ces nanoémulsions antérieures arrivent de manière non spécifique, jusqu'à la zone tumorale, typiquement par mécanisme de diffusion désigné EPR et connu de l'homme du métier (décrit par exemple dans H. Maeda, J. Wu, T. Sawa, Y. Matsumura, K. Hori, Tumor vascular permeability and the EPR effect in macromolecular therapeutics : A review, J. Control. Release, 65 (2000) 271-284) : les nanosystèmes encapsulant les médicaments arrivent par diffusion sanguine jusqu'aux zones tumorales qui sont très vascularisées. Le document WO 95/33494 décrit des nanoémulsions pour IRM comprenant une phase aqueuse, une phase lipidique comprenant une huile telle que l'huile de carthame et un tensioactif comprenant un chélate de métal paramagnétique amphiphile et un lipide amphiphile tel que la lécithine.

Les nanoémulsions du demandeur sont très avantageusement biovectorisées car destinées au contraire à l'imagerie moléculaire diagnostique : les nanogouttelettes de la nanoémulsion présentent, incorporés au niveau de la couche formée par les surfactants, un ou plusieurs biovecteurs ou ligands de ciblage spécifiques qui viennent reconnaître spécifiquement par interaction moléculaire (affinité cible/ligand) la cible biologique (récepteur, enzyme ...) dont l'expression est modifiée dans la zone pathologique. Ces ligands de ciblage sont également désignés pharmacophores ou ligands de reconnaissance par l'homme du métier.

Or un problème technique très difficile à résoudre est précisément d'incorporer de manière appropriée et stable dans la durée un ou plusieurs biovecteurs de ciblage pour l'imagerie moléculaire, en quantité suffisante pour obtenir une spécificité du marquage, mais pas trop élevée pour éviter des prix de revient industriels trop élevés.

Au vu de cet art antérieur complexe, on voit la difficulté d'obtenir des nanoémulsions vectorisées pour IRM, à la fois chimiquement industrialisables et stables, et biologiquement performantes. Un raisonnement consistant à partir notamment du document US 2007/0148194 et dire qu'il suffisait de faire varier des teneurs en tensioactifs serait un raisonnement *a posteriori* une fois l'invention identifiée, de nombreuses possibilités s'étant en fait présentées à l'homme du métier pour améliorer l'art antérieur.

Le demandeur a réussi à obtenir des nanoémulsions de lanthanides, sous forme de nanogouttelettes vectorisées et résolvant les problèmes techniques de l'art antérieur. En particulier le demandeur a réussi à sélectionner des compositions optimisées comprenant suffisamment de surfactant pour stabiliser la taille des nanoparticules, mais pas trop pour éviter l'incorporation insuffisante des biovecteurs. Dans les émulsions essentiellement thérapeutiques de l'art antérieur, le composé thérapeutique est essentiellement encapsulé à l'intérieur de la nanogouttelette, souvent en mélange avec les glycérides de l'huile. Dans les nanoémulsions du demandeur, le ligand de reconnaissance doit pouvoir se loger au sein de l'interface huile/eau, en s'ancrant dans la membrane/pellicule amphiphile des surfactants. Il n'était pas du tout évident pour l'homme du métier de trouver les bons composés et les bons rapports de quantités entre les surfactants, l'huile, et les biovecteurs, qui permettent d'obtenir des nanoémulsions efficaces en imagerie moléculaire et sans une perte de biovecteurs très onéreux.

A cet effet, l'invention concerne selon un premier aspect une composition de nanoémulsion huile dans eau pour IRM, comprenant :

- une phase aqueuse, représentant 70 à 90% en poids de la composition, avantageusement 75 à 85%, plus avantageusement de 78 à 82%
- une phase lipidique comprenant une huile, représentant 9.5 à 29.5% en poids de la composition, avantageusement 14 à 25%, plus avantageusement 17 à 21%,
- un tensioactif à l'interface entre les phases aqueuse et lipidique, le tensioactif comprenant au moins un chélate de métal paramagnétique amphiphile, au moins un ligand de ciblage amphiphile et éventuellement un lipide amphiphile ;

    - la teneur totale en tensioactif en poids par rapport à l'huile étant comprise entre 4 et 10%, avantageusement entre 5 et 8 % ;
    - la teneur totale en tensioactif en poids par rapport à la composition étant comprise entre 0.35 et 2.95%, avantageusement entre 0.5 et 2 % ;
    - l'huile comprenant au moins 70%, avantageusement au moins 80%, de façon avantageuse au moins 95% en poids, notamment au moins 97 %, d'acides gras saturés en C6-C18, avantageusement en C6-C14, plus avantageusement en C6-C10.

Très avantageusement la phase lipidique est constituée par l'huile. Le tensioactif comprend au moins un biovecteur amphiphile de ciblage, également désigné pharmacophore ou ligand de ciblage amphiphile.

La nanoémulsion ne comprend pas de nanocristaux métalliques. Les acides gras saturés se trouvent avantageusement sous la forme de triglycérides d'acides gras saturés. L'huile comprend au moins 70%, de préférence au moins 80, 90, 95, 97 % d'acides gras saturés en C6-C10.

L'homme du métier comprend que le tensioactif (surfactant) à l'interface est représenté par l'ensemble des tensioactifs utilisés, c'est-à-dire comme expliqué en détail dans la demande : des lipides amphiphiles présents ou non selon les réalisations, des molécules de chélates amphiphiles, des biovecteurs amphiphiles, et le cas échéant d'autres composés tels que des dérivés pégylés (lipides couplés à des PEG). De par leur structure amphiphile, les molécules de biovecteurs amphiphiles jouent un rôle de tensioactif étant précisé que leur quantité est faible par rapport aux autres composés amphiphiles utilisés.

Il est précisé que compte tenu notamment du volume injectable aux patients, de l'ordre de 10 à 50 ml, l'huile est utilisée à un taux suffisamment élevé, d'au moins 9.5%, afin d'avoir une solution suffisamment concentrée et un signal en IRM suffisant. Il est nécessaire d'avoir une concentration adaptée pour la durée d'injection, le moment de l'acquisition du signal et le traitement associé des données par le praticien. Une solution trop diluée la rendrait inutilisable pour les examens d'imagerie médicale. La concentration en émulsion de la composition diagnostique injectée au patient

est avantageusement comprise entre 0.1 à 20 ml/Kg de poids corporel. Pour un volume d'agent de contraste injecté de l'ordre de 5 à 50 ml, la concentration de l'agent de contraste est de l'ordre de 0.1 à 20 ml/Kg de poids corporel, notamment 1 à 10 et typiquement 5 ml/Kg de poids corporel. La quantité de chélate de lanthanide est de l'ordre de 1 à 100 μMol Gd/Kg notamment 1 à 10 μMol Gd/Kg, ce qui permet d'obtenir une bonne qualité du signal en IRM

Les produits (nanoémulsions) vectorisés du demandeur ont une taille des particules suffisamment petite pour leur permettre de circuler dans les milieux biologiques sans dégradation du produit, jusqu'à la cible du ligand biovecteur fixé aux goutellettes. La taille est typiquement de 30 à 300 nm, avantageusement 50 à 250 nm, notamment 100 à 200 nm, en particulier 150 à 200 nm.

Les nanogouttelettes comprennent chacune un nombre de biovecteurs de l'ordre de 100 à 5000, notamment 500 à 3000, notamment 1800 à 2500 (par exemple 2000) ce qui permet le ciblage efficace selon l'affinité et la multivalence du biovecteur. Les résultats biologiques obtenus grâce aux nouvelles nanoémulsions du demandeur montrent de plus que les biovecteurs sont avantageusement répartis sur l'ensemble de la surface externe des nanogouttelettes, ce qui se traduit par une multivalence optimisée des biovecteurs.

Les biovecteurs amphiphiles représentent avantageusement 0.01 à 10% en poids du total des tensioactifs, avantageusement 0.05 à 5%, notamment 0.05 à 1 %. Le produit de contraste injecté ayant les compositions de nanoémulsions décrites ont une affinité avantageusement de l'ordre de 0.1 à 100 nM, notamment 1 à 50 nM, avantageusement 1 à 10 nM (l'affinité par biovecteur amphiphile, autour de 0.1 à 100 μM est multipliée par le nombre de biovecteurs par nanoparticule).

Avantageusement la composition comprend 0.001 à 0.1 % en poids de biovecteur amphiphile, notamment 0.01 à 0,1%.

Les nanoémulsions du demandeur ont en outre l'avantage de pouvoir contrôler le type et la quantité de biovecteurs, et notamment de pouvoir incorporer des biovecteurs différents. Par exemple une nanogouttelette comprendra :

- un biovecteur amphiphile qui permet l'accession à une zone physiologique pathologique, par exemple un biovecteur de passage à travers la BHE (barrière hémato encéphalique)
- un autre biovecteur amphiphile de ciblage qui permet ensuite le ciblage d'un marqueur biologique cible surexprimé par certaines cellules de cette zone pathologique

Les interactions moléculaires entre le biovecteur de ciblage et le marqueur biologique cible permettent la captation des nanogouttelettes au niveau de la zone pathologique, et l'imagerie IRM qui en résulte permet de localiser précisément la zone pathologique.

On entend par le terme « acide gras » désigner des acides carboxyliques aliphatiques présentant une chaîne carbonée d'au moins 6 atomes de carbone. Les acides gras naturels possèdent une chaîne carbonée de 4 à 28 atomes de carbone (généralement un nombre pair). On parle d' « acide gras à longue chaîne » pour une longueur de 14 à 22 carbones et à très longue chaîne s'il y a plus de 22 carbones. On parle au contraire d' « acide gras à courte chaîne » pour une longueur de 6 à 10 carbones, en particulier 8 ou 10 atomes de carbone. L'homme du métier connaît la nomenclature associée et en particulier utilise :

- Cn-Cp pour désigner une fourchette d'acides gras en Cn à Cp
- et Cn+Cp, le total des acides gras en Cn et des acides gras en Cp

Par exemple :

- les acides gras entre 14 et 18 atomes de carbone s'écrivent « acides gras en C14-C18 »
- le total des acides gras en C16 et des acides gras en C18 s'écrit C16 +C18

Très avantageusement, l'huile comprend moins de 10%, de préférence moins de 5% d'acides gras insaturés, en particulier moins de 5%, et de préférence moins de 2%, moins de 1% d'acides gras insaturés en C14-C18 ou en C14-C22.

Par exemple l'huile est le MIGLYOL®

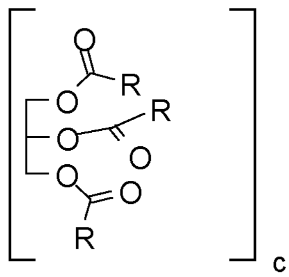

(R = $C_8$ + $C_{10}$) >95%

P03040

ou l'un des ses dérivés connus, par exemple le MIGLYOL® 810 or MIGLYOL® 812 (caprylic/capric triglyceride), MIGLYOL® 818 (caprylic/capric/linoleic triglyceride), le MIGLYOL® 612 (glyceryl trihexanoate), d'autres dérivés MIGLYOL® propylène glycol dicaprylate dicaprate.

Par exemple le Miglyol® 812 a la composition suivante :

| | |
|---|---|
| Acide Caproïque ($C_{6\text{-}0}$) : | max 2 % |
| Acide Caprylïque ($C_{8\text{-}0}$) : | 50 à 65 % |
| Acide Caprique ($C_{10\text{-}0}$) : | 30 à 45 % |
| Acide Laurique ($C_{12\text{-}0}$) : | max 2 % |
| Acide Miristique ($C_{14\text{-}0}$) : | max 1 % |
| Acide Linoleique ($C_{18\text{-}2}$) : | - |

Selon des variantes, l'huile saturée est un mélange d'huiles saturées comprenant chacune au moins 70%, de préférence au moins 80, 90, 95% d'acides gras saturés de 6 à 10 atomes de carbone.

On rappelle que le terme tensioactif ou surfactant fait référence à des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

De préférence, les acides gras saturés des huiles saturées utilisées par le demandeur sont utilisés sous forme de mono, di ou triglycérides, de préférence triglycérides.

De manière préférée, l'huile des émulsions du demandeur comprend des acides gras saturés dans les variantes suivantes :

- C6-C18 > 70%, de préférence C6-C18 > 80% de préférence C6-C18 >95%, et encore de préférence C6-C18 > 98%
- C6-C14 > 70%, de préférence C6-C14 > 80% de préférence C6-C14 >95%, et encore de préférence C6-C14 > 98%
- C8+C10 > 70%, de préférence C8+C10 > 80% de préférence C8+C10 >95%, et encore de préférence C8+C10 > 98%
- C8 compris entre 40 et 70% de préférence 50 à 65% et/ou C10 compris entre 20 et 50% de préférence 30 à 45%, le total C8+C10 étant supérieur à 80%.

Selon des réalisations préférées, la nanoémulsion lipidique a pour composition en poids :

1) 70 à 90% en poids de phase aqueuse, avantageusement 75 à 85%, plus avantageusement de 78 à 82%

2) 9.5 à 29.5% en poids de phase lipidique comprenant une huile, avantageusement 14 à 25%, plus avantageusement 17 à 21 %;

3) 0.38 à 2.95% de tensioactif (c'est à dire 4 à 10% de la phase lipidique), le tensioactif comprenant 50 à 95% en poids de lipide amphiphile, 5 à 50 % en poids de chélate de métal paramagnétique amphiphile, avantageusement 5 à 30% en poids de chélate de métal paramagnétique amphiphile, et 0.05 à 7%, préférentiellement 0.05 à 5% en poids de biovecteur amphiphile de ciblage.

Avantageusement le chélate amphiphile est un chélate macrocyclique choisi parmi : DOTA, DO3A, HPDO3, BTDO3A, PCTA et tout dérivé connu de ces chélates, notamment décrit par exemple dans Mini Reviews in Medicinal Chemistry, 2003, vol 3, n°8.

Selon des réalisations préférées, la nanoémulsion lipidique a pour composition en poids :

1) 70 à 90% en poids de phase aqueuse, avantageusement 75 à 85%, plus avantageusement de 78 à 82%

2) 9.5 à 29.5% en poids de phase huileuse, avantageusement 14 à 25%, plus avantageusement 17 à 21 %;
3) 0.38 à 2.95% de tensioactif, le tensioactif comprenant 95 à 99.95 % de chélate amphiphile et 0.05 à 5% de biovecteur amphiphile de ciblage.

Dans cette réalisation, le chélate assure un rôle de tensioactif suffisant permettant de ne pas utiliser de lipide amphiphile tensioactif. Le chélate est alors avantageusement le chélate PCTA amphiphile ou l'un de ses dérivés connus, notamment décrit dans WO2006100305 en particulier les composés de formule I page 52 à 55 de ce document.
Selon des réalisations préférées, la nanoémulsion lipidique a pour composition en poids :

1) 70 à 90% , de préférence 75 à 85%, avantageusement 78 à 82%, notamment 79 à 81 % de phase aqueuse
2) 9.5 à 29.5%, de préférence 14 à 25%, avantageusement 17 à 21% d'huile, l'huile comprenant au moins 70%, de préférence au moins 80, 90, 95% d'acides gras saturés C6-C14, de préférence C6-C10
3) 0.38 à 2.95%, avantageusement 0.5 à 1.5% de tensioactifs totaux

étant précisé que les total des pourcentages de 1), 2) et 3) est égal à 100% ; Selon des réalisations préférées, les tensioactifs totaux comprennent :

3.1) 0 à 90% de lipides amphiphiles
3.2) 10 à 100 %, avantageusement 10 à 40% de chélates amphiphiles
3.3) 0.01 à 10%, avantageusement 0.05 à 5% de biovecteurs amphiphiles
3.4) 0 à 30% de dérivé amphiphile pégylé.

En particulier, les réalisations suivantes sont avantageuses :

| % en poids de phase aqueuse (1) | % en poids d'huile (2) | % en poids de tensioactif par rapport à l'huile (3) | % en poids de tensioactif par rapport à la composition totale (4) |
|---|---|---|---|
| 70-90 | 9.5 - 29.5 | 4 à 10 % de (2) | [0.38 - 2.95] % (*) |
| 75-85 | 14-25 | 4 à 10 % de (2) | [0.56 - 2.5] % |
| 78-82 | 17-21 | 4 à 10 % de (2) | [0.68 - 2.1] % |
| 75-85 | 14-25 | 5 à 8 % de (2) | [0.7 - 2] % |
| 78-82 | 17-21 | 5 à 8 % de (2) | [0.85 - 1.68] % |
| Etant précisé que le total (1) + (2) + (3) = 100%<br>(*) la gamme [0.38 - 2.95] correspond à 0.04*9.5 = 0.38 % et 0.1*29.5= 2.95 | | | |

Ces gammes sont préférées notamment dans la mesure où elles permettent d'obtenir une taille de nanoparticules comprise entre 150 et 300 nm, et en particulier autour de 150 à 200 nm. La taille et la stabilité des particules sont très satisfaisantes, ainsi que la viscosité (de l'ordre de 2 à 3 mPa.s). Leur comportement est newtonien, ce qui constitue un avantage important pour des solutions pharmaceutiques injectables.
Le demandeur a pu constater qu'au delà de 30% d'huile dans la composition, celle-ci adopte un comportement trop rhéofluidifiant et/ou une viscosité (la viscosité devenant alors supérieure à des valeurs de 4 à 5 mPa.s) non adaptées pour l'injection intraveineuse.
De plus les formulations obtenues sont iso-osmolaires, ce qui évite des désagréments pour le patient lors de l'injection. En outre, la quantité de chélates de lanthanides et la quantité de biovecteurs greffés aux nanoparticules sont très bien adaptées pour l'imagerie IRM. La composition est de plus capable de supporter la stérilisation à la chaleur, typiquement par autoclavage.
L'invention concerne aussi un agent de contraste comprenant une composition telle que décrite précédemment.
On a par exemple les gammes de proportions suivantes des constituants.

| Phase aqueuse de la composition (a) | Phase lipidique (huile + tensioactif) en % de la composition (b) | Teneur (%) en tensioactifs de l'huile (c) | Teneur % en lipides amphiphiles des tensioactifs | Teneur % en chélates amphiphiles des tensioactifs | Teneur % en lipides pégylés des tensioactifs | Teneur % en biovecteurs amphiphiles des tensioactifs |
|---|---|---|---|---|---|---|
| 75 à 85, de préférence 78 à 82, de préférence 80 | 14 à 25, de préférence 17 à 21, de préférence 20 | 5 à 10, de préférence 5 à 8, de préférence 6 | 50 à 95 | 5 à 25 | 0 | 0.05 à 5 |
| 75 à 85, de préférence 78 à 82, de préférence 80 | 14 à 25, de préférence 17 à 21, de préférence 20 | 5 à 10, de préférence 5 à 8, de préférence 6 | 75 à 95 | 5 à 25 | 5 à 15 | 0.05 à 5 |
| 75 à 85, de préférence 78 à 82, de préférence 80 | 14 à 25, de préférence 17 à 21, de préférence 20 | 5 à 10, de préférence 5 à 8, de préférence 6 | 0 | 95 à 99.95 | 0 à 5 | 0.05 à 5 |

Le total dans le tensioactif des teneurs en lipides amphiphiles, chélates amphiphiles, lipides pégylés, biovecteurs amphiphiles, est de 100%.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement lipophile par une fonction éther ou ester tels que les esters de sorbitan comme par exemple les monooléate et monolaurate de sorbitan ; les lipides polymérisés ; les esters de sucre tels que les mono-et di-laurate, mono-et di-palmitate, mono- et distéarate de saccharose; lesdits tensioactifs pouvant être utilisés seuls ou en mélanges.

Le lipide amphiphile réactif est incorporé dans la couche formée à l'interface stabilisant la phase dispersée, où il est susceptible de se coupler par exemple avec un composé réactif présent dans la phase aqueuse. Avantageusement le lipide amphiphile est un phospholipide, de préférence choisi parmi : phosphatidylcholine (appelée aussi lécithine), dioléoylphosphatidylcholine, dimyristoylphosphatidylcholine, dipalmitoylphosphatidylcholine, distéaroylphosphatidylcholine, phosphatidyléthanolamine, sphingomyéline, phosphatidylsérine, phosphatidylinositol. La lécithine est un lipide amphiphile préféré.

Avantageusement le lipide amphiphile est un lipoïde, notamment l'EPC (Ethyl Phospho Choline et ses dérivés connus notamment de Avanti Polar Lipids) ou le lipoide S75

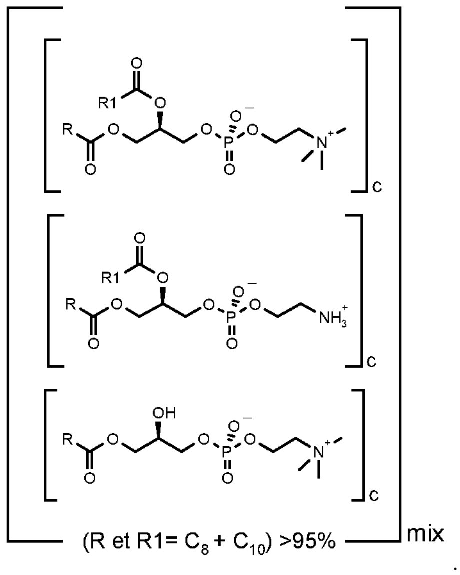


| | |
|---|---|
| Phosphatidylcholine (+LPC): | 68 à 73 % |
| Phosphatidylethanolamine : | 7 à 10 % |
| Lysophosphatidylcholine : | < à 3 % |
| Phosphore : | 3,4 à 3,7 % |

Selon un mode de réalisation particulier, tout ou partie du lipide amphiphile peut posséder une fonction réactive, telle qu'un groupe maléimide, thiol, amine, ester, oxyamine ou aldéhyde. La présence de fonctions réactives permet le greffage de composés fonctionnels au niveau de l'interface.

On pourra utiliser pour la phase amphiphile, en plus du chélate et du lipide amphiphile, de manière non obligatoire, et en particulier afin d'agir sur le caractère furtif du produit dans l'organisme, des lipides pégylés c'est-à-dire porteurs de groupes oxyde de polyéthylène (PEG), tels que le polyéthylèneglycol/phosphatidyl-éthanolamine (PEG-PE). Par « polyéthylèneglycol» PEG, au sens de la présente demande, on désigne de façon générale, des composés comprenant une chaîne -CH2-(CH2-O-CH2)k-CH2OR3 dans laquelle k varie de 2 à 100 (par exemple 2, 4, 6, 10, 50), et R3 est choisi parmi H, alkyle ou -(CO)Alk, le terme "alkyle" ou "alk" désignant un groupe aliphatique hydrocarboné, linéaire ou ramifié, ayant environ de 1 à 6 atomes de carbone dans la chaîne. Le terme « polyéthylèneglycol » tel qu'employé ici englobe notamment les composés aminopolyéthylèneglycols. On citera notamment les PEG 350, 750, 2000, 3000, 5000, modifiés par ajout de groupements amphiphiles pour s'insérer au sein de la couche de tensioactif de la nanoparticule, notamment :

- 1,2-Distearoyl-sn-Glycero-3-Phosphoethanolamine-N-[Methoxy(Polyethylene glycol)-350]
- 1,2-Distearoyl-sn-Glycero-3-Phosphoethanolamine-N-[Methoxy(Polyethylene glycol)-550],
- 1,2-Distearoyl-sn-Glycero-3-Phosphoethanolamine-N-[Methoxy(Polyethylene glycol)-750]

On utilisera notamment le lipide pégylé :

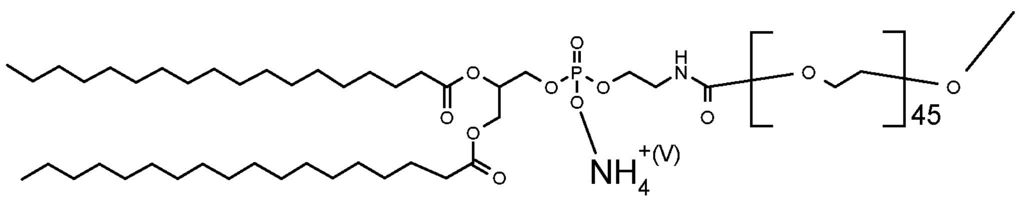

La phase aqueuse est avantageusement de l'eau ou une solution aqueuse pharmaceutiquement acceptable telle qu'une solution saline, une solution tampon.

Par « chélate amphiphile », on entend que le chélate a été modifié chimiquement de manière à présenter une

lipophilie (une lipophilie suffisamment élevée, ou à l'inverse une hydrophilie suffisamment faible), telle qu'il puisse s'ancrer au sein de la couche de tensioactifs de la nanoparticule et de manière à former une composition lipidique suffisamment stable pour une utilisation diagnostique satisfaisante. On aura par exemple, de manière non limitative, un choix des groupements amphiphiles greffés au chélate tel que la valeur HLB (la balance hydrophile / lipophilie) du chélate soit de l'ordre de 12 à 20 pour les chélates ancrés aux nanoémulsions lipidiques.

En plus du lipide amphiphile, les chélates amphiphiles utilisés par le demandeur jouent avantageusement le rôle de tensioactif, tout en ayant l'avantage d'apporter une quantité très élevée d'entités signal à la nanoparticule. Avantageusement, le nombre de chélates de lanthanide par nanogouttelette est d'au moins 1000 et typiquement au moins 5000, 10 000, 20 000, 50 000 à 100 000.

On décrit plus précisément à titre d'exemples des chélates pouvant être utilisés, dans la mesure ou comme décrit plus haut ils comprennent au moins un groupe amphiphile d'ancrage à la nanoparticule lipidique. Le demandeur décrit les chélates utilisables, étant précisé que les chélates particulièrement avantageux pour ses nouvelles émulsions sont des chélates macrocycliques. En effet, des nanosystèmes lipidiques utilisant des chélates macrocycliques sont significativement moins exposés que les chélates linéaires à un risque de transmétallation au zinc en particulier, qui s'accompagne d'un risque de libération non souhaitée de lanthanide, en particulier du gadolinium Gd3+ toxique.

On pourra utiliser les chélates macrocycliques notamment de formule suivante (illustration avec le gadolinium Gd, d'autres lanthanides étant aussi appropriés)

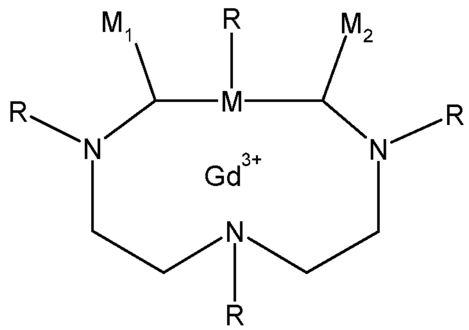

avec :

M-M1-M2 forme un noyau pyridine
ou M1 et M2 sont absents et M représente une liaison
ou M est N-R et M1 et M2 représentent un atome d'hydrogène ou un méthyle
avec R est choisi indépendamment parmi $CH_2CO_2$- ou H ou CHX-$CO_2$- , avec au moins un R étant $CHXCO_2$- et X étant L-B ;

On pourra utiliser notamment un chélate macrocyclique parmi 1,4,7,10-tetraazacyclododecan-1,4,7,10-tetraacetic acide (DOTA), 1,4,7,10-tetraazacyclododecan-1,4,7-triacetic acide (DO3A), 10-(2-hydroxypropyl)-1,4,7,10-tetraazacyclododecan-1,4,7-triacetic acide (HPDO3A), (MCTA), (DOTMA), 3,6,9,15-tetraazabicyclo[9.3.1]pentadeca-1(15),11,13-triene-3,6,9-triacetic acide (PCTA).

On pourra aussi utiliser des dérivés dans lesquels un ou plusieurs groupes carboxyliques sont sous forme d'un sel, ester, amide correspondant ; ou un composé correspondant dans lequel un ou plusieurs groupes carboxyliques sont remplacés par un groupe phosphonique et/ou phosphinique.

On peut aussi utiliser un chélate parmi : DOTA gadofluorines, DO3A, HPDO3A, TETA, TRITA, HETA, DOTA-NHS, M4DOTA, M4DO3A, PCTA et leurs dérivés .

On pourra utiliser un chélate linéaire connu choisi parmi EDTA, DTPA diéthylènetriaminopentaacétique acide, N-[2-[bis(carboxyméthyl)amino]-3-(4-ethoxyphenyl)propyl]-N-[2-[bis(carboxyméthyl)amino]éthyle]-L-glycine (EOB-DTPA), N,N-bis[2-[bis(carboxyméthyl)amino]éthyle]-L-glutamique acide (DTPA-GLU), N,N-bis[2-[bis(carboxyméthyl)amino]éthyle]-L-lysine (DTPA-LYS), des dérivés mono- ou bis-amide du DTPA, tels que N,N-bis[2-[carboxyméthyl[(méthylcarbamoyl)méthyle]amino]éthyle] glycine (DTPA-BMA), 4-carboxy-5,8,11-tris(carboxyméthyl)-1-phenyl-2-oxa-5,8,11-triazatridecan-13-oic acide (BOPTA),

De manière plus large, le ou les chélates formant l'entité signal pourront répondre à la formule du document WO 01/60416 ou WO 03/062198 (page 23 à 25).

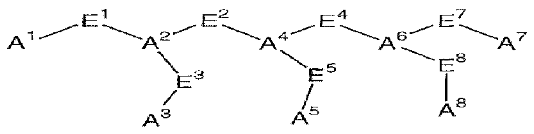

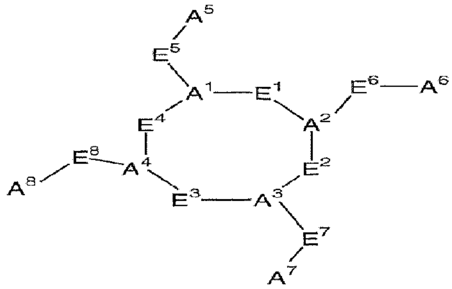

On pourra utiliser en particulier les composés DOTA, NOTA, DO3A, AAZTA, HOPO, ainsi que leur multimères et dérivés connus notamment :

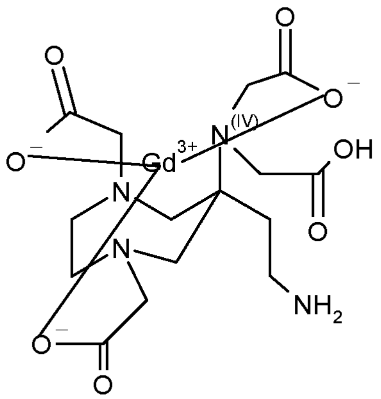

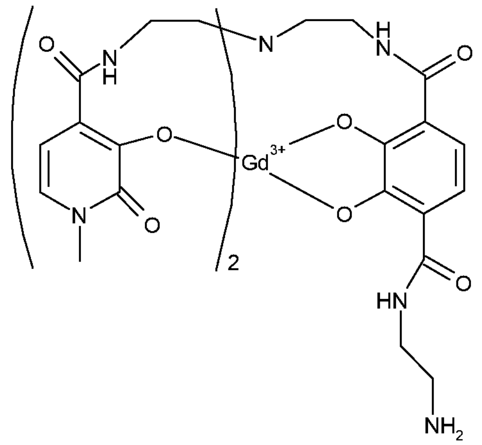

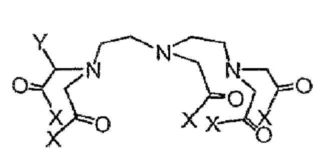,
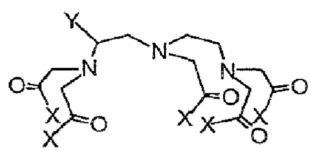,
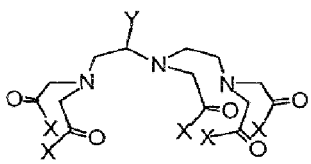,

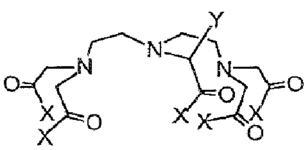,
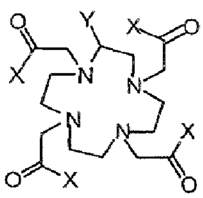,
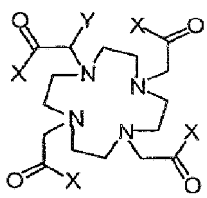,
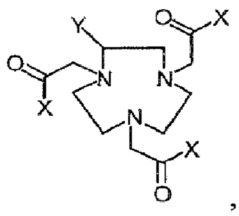,

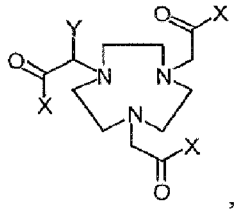, 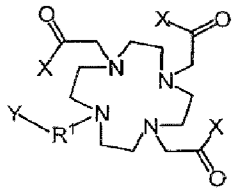, 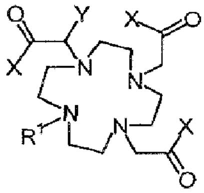, 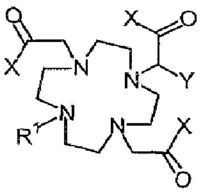,

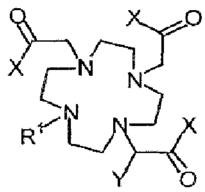, 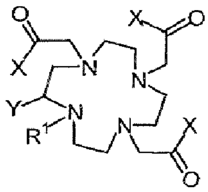, 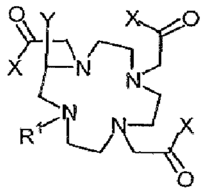, 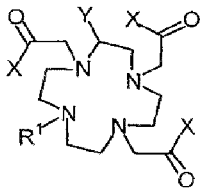,

and

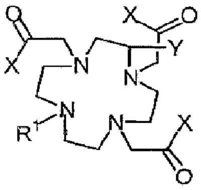,

avec X un groupe capable de coordiner un cation métallique, de préférence O-, OH, $NH_2$, $OPO_3$-, NHR avec R une chaîne aliphatique.

On citera aussi les chélates rappelés dans WO 03/011115 pages 8 à 11.

Comme exemples de macrocycles amphiphiles très avantageux, on peut citer les structures suivantes dérivées des coeurs PCTA et DOTA.

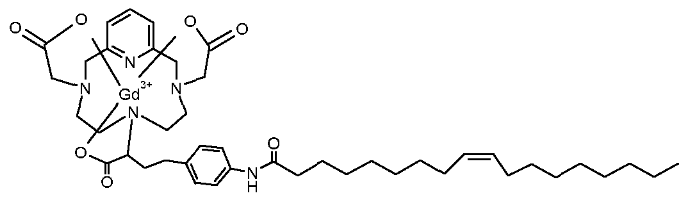

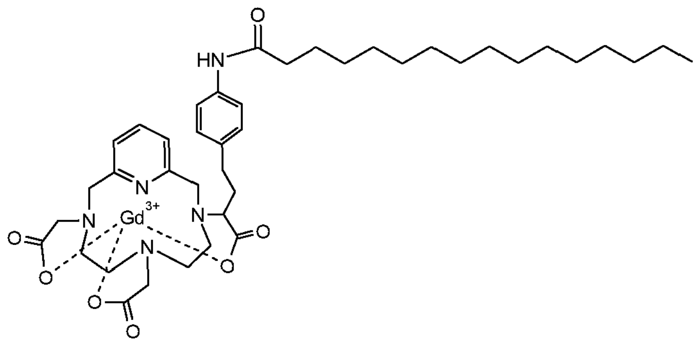

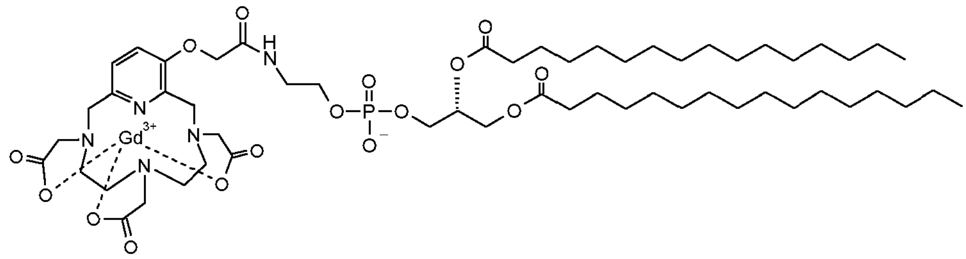

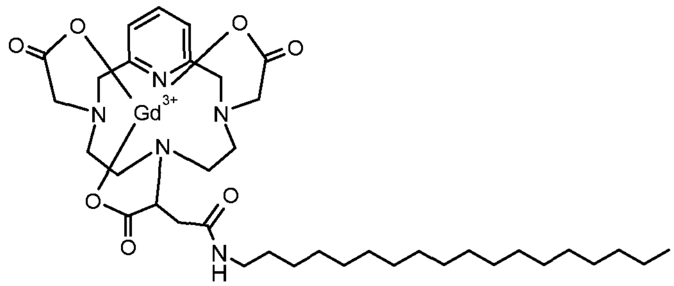

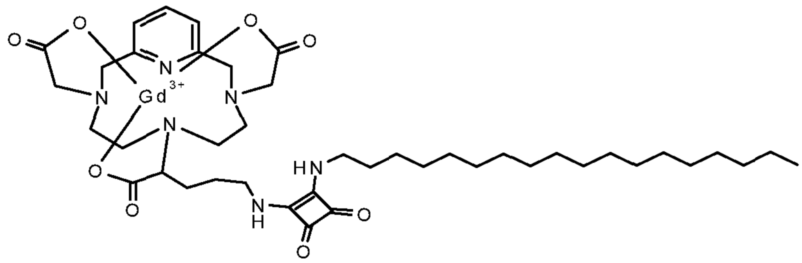

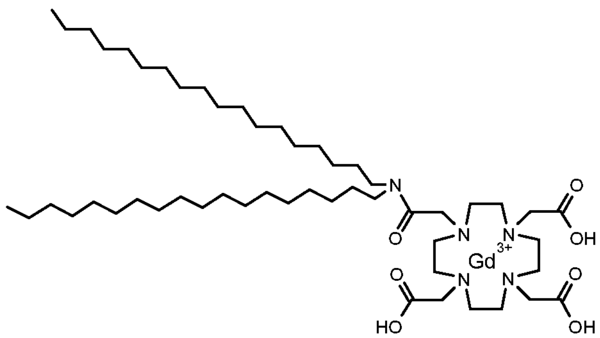

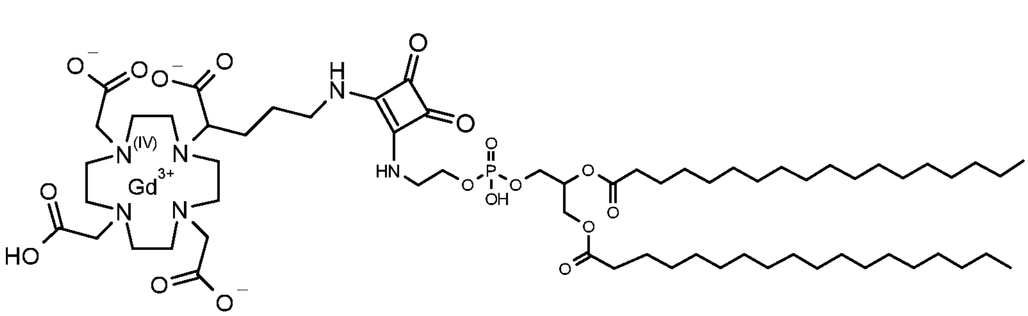

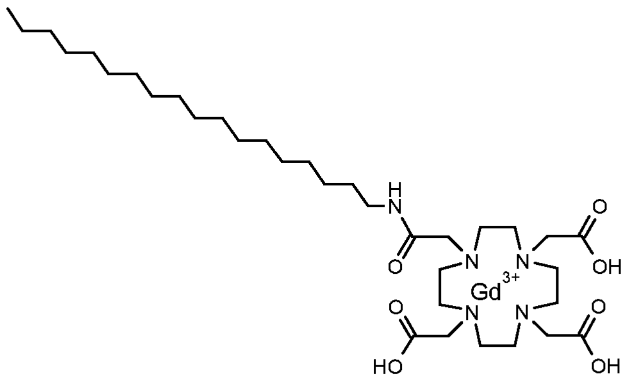

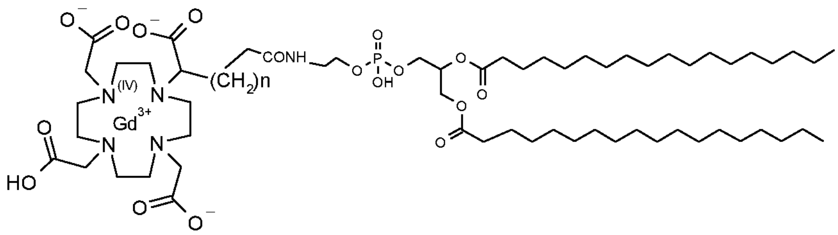

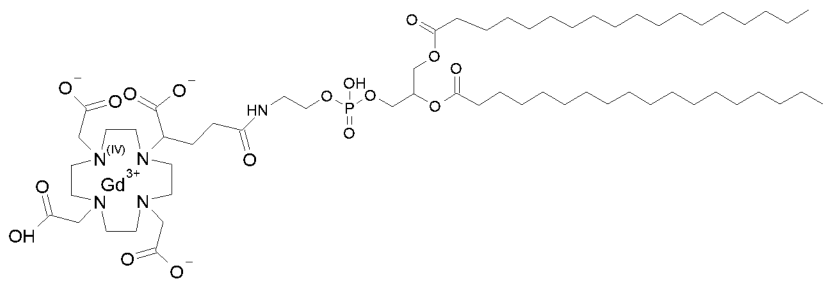

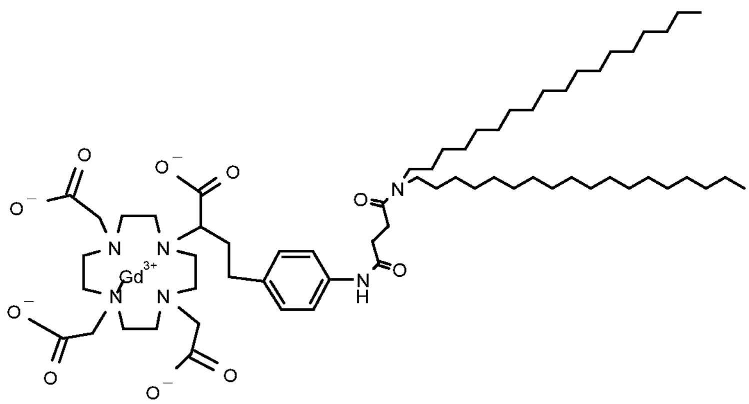

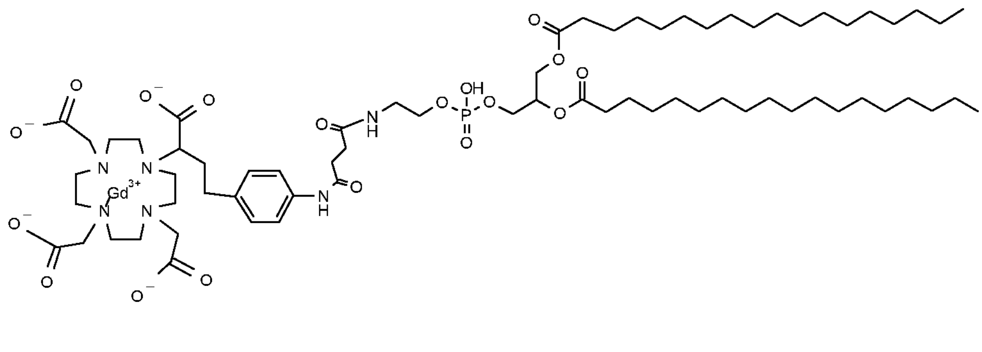

Les formules sont présentées dans la demande notamment dans les exemples détaillés avec des groupes de liaison entre le chélate et la chaîne carbonée lipophile. Un grand nombre de groupes de liaison sont uilisables, par exemple : rien ou une simple liaison, les groupes alkyle ou alkylène C1-10, par exemple C1-6 alkylène, PEG par exemple CH2-(CH2-O-CH2)k-CH2 avec k =1 à 50 notamment là 10, $(CH_2)_3$-NH, NH-$(CH_2)_2$-NH, NH-$(CH_2)_3$-NH, , $(CH_2)_n$, $(CH_2)_n$-CO-, -$(CH_2)_n$NH-CO- avec n = 2 à 10 , $(CH_2CH_2O)_q(CH_2)_r$-CO- , $(CH_2CH_2O)$q$(CH_2)_r$-NH-CO- avec q = 1-10 et r=2-10, $(CH_2)_n$-CONH - , $(CH_2)_n$-CONH - PEG, $(CH_2)_n$-NH-HOOC-$CH_2$-O-$(CH_2)_2$-O-$(CH_2)_2$-O-$CH_2$-COOH ; HOOC-$(CH_2)_2$-$CO_2$-$(CH_2)_2$-OCO-$(CH_2)_2$-COOH ; HOOC-CH(OH)-CH(OH)-COOH; HOOC-$(CH_2)_n$-COOH; $NH_2$-$(CH_2)_n$-$NH_2$, avec n=0-20; $NH_2$-$(CH_2)_n$-$CO_2$H; $NH_2$-$CH_2$ - ($CH_2$-O-$CH_2$)$_n$-$CO_2$H avec n=1 à 10, P1-1-P2, identiques ou différents, P1 et P2 étant choisis parmi O, S, NH, rien, $CO_2$, NHCO, CONH, NHCONH, NHCSNH, $SO_2$NH-, $NHSO_2$-, squarate

avec 1 = alkyle, alkoxyalkyle, polyalkoxyalkyle (PEG), alkyle interrompu par un ou plusieurs squarates ou par un ou plusieurs aryles, avantageusement phényles, alcényle, alcynyle, alkyle interrompus par un ou plusieurs groupes choisis parmi -NH-, -O-, -CO-, -NH(CO)-, - (CO)NH-, -O(CO)-, ou -(OC)O-).

Comme exemples de dérivés DTPA amphiphiles, on utilisera ceux des exemples détaillés ou d'autres tels que :

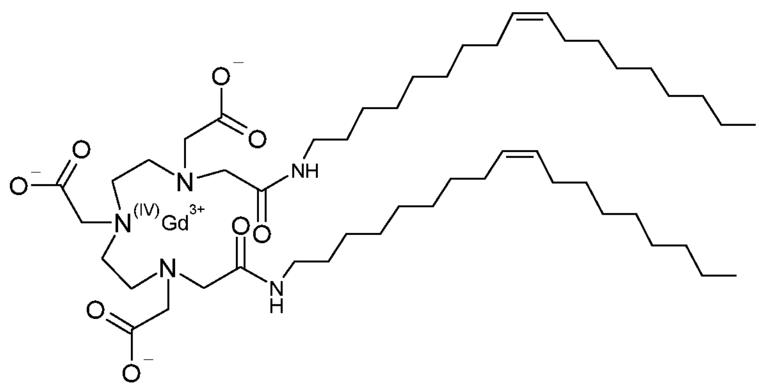

On rappelle que pour obtenir des chélates macrocycliques particulièrement préférés pour les nouvelles nanoémulsions, du type DO3A, BT-DO3A, HP-DO3A,DOTA, DOTAM, DOTMA, DOTA-GA, et autres chélates macrocycliques porteurs de chaînes carbonées, on utilisera par exemple le 1,4,7,10-tetraazacyclododécane ou dérivés, préparés comme cela est connu de l'homme du métier, à partir de la diéthylène triamine ou d'autres dérivés polyazotés linéaires.

On rappelle que le 1,4,7,10-tetraazacyclododécane est habituellement obtenu à partir de dérivés bicycliques ou de composés tétracycliques (tels que le 2a,4a,6a,8a-décahydrotétraazacyclopenta[fg]acénaphthylène).

Ces composés tétracycliques sont eux-même obtenus typiquement dans un procédé comprenant une étape d'addition à la diéthylène triamine d'agents connus tels que le benzotriazole ou des composés (R1R2) CH- X - CH (R3R4), des composés mono ou dicarbonylés R1C(=O) - C(=O)R2, des composés CSNH2 - CSNH2, avec R1 à R4 étant notamment H, OH, CH3, un alkyle en C1-C3, un halogène.

Cette addition conduit à des composés à trois cycles connus tels que le 3H,6H-2a,5,6,8a-Octahydro-tétraazaacénaphthylène, obtenu par exemple à partir du glyoxal (et décrit notamment dans Tetrahedron Letters, vol 22, n°18, 1980, pp1711-1714), composés à trois cycles auxquels sont ensuite greffés des agents bialkylant variés [X1 - A - X1], comprenant typiquement deux groupes partants.

Les agents bialkylants connus sont typiquement le dichloroéthane ou le dibromoéthane. Le demandeur a d'ailleurs constaté qu'il était très avantageux d'utiliser des agents bialkylants [X1 - A - X2] comprenant des groupes partants X1 et X2 différents (halogènes, tosyl, mesyl etc ...), tels que le bromo chloro éthane, le bromo chloro propane. En effet, le rendement de la réaction de bialkylation est significativement amélioré à l'échelle industrielle.

Un procédé de préparation très avantageux de macrocycles polyazotés dont le 1,4,7,10-tetraazacyclododécane et le 1,4,8,11-tétraaza cyclotétradécane (cyclam), est un procédé comprenant les étapes successives suivantes :

1) addition à un composé à trois cycles azotés fusionnés,
notamment à l'un des composés suivants :

- 3H,6H-2a,5,6,8a-Octahydro-tétraaza-acénaphthylène,
- octahydro-1,3a,6a,9-tetraazaphénalène,
- 5a,8b diméthyl-octahydro-2a,5,6,8a-tetraaza-acénaphtylène
- 9a,9b-diméthyl-octahydro-1,3a,6a,9-tetraazaphénalène
- octahydro-2a,5,6,8a- tetraaza-acénaphtylène

d'un agent bialkylant [X1 - A - X2] comprenant deux groupes partants X1 et X2 différents et choisis de préférence parmi halogène, tosyl, mesyl, A étant de préférence un alkylène linéaire ou ramifié, de préférence A étant CH2-CH2, X1 et X2 étant de préférence un halogène Cl ou Br, par exemple [X1 - A - X2] étant ClCH2- CH2Br pour obtenir des composés à quatre cycles azotés fusionnés, par exemple les composés :

- 2a,4a,6a,8a- décahydrotétraazacyclopenta[fg]naphtylène
- 8b-méthyl 2a,4a,6a,8a- décahydrotétraazacyclopenta[fg]acenaphthylène
- 8b,8c diméthyl 2a,4a,6a,8a- décahydrotétraazacyclopenta[fg]acenaphthylène
- 9b,9c-diméthyl décahydro - 2a,4a,7a,9a tetraaza cyclopenta [cd]phénalène
- décahydro - 2a,4a,6a,8 a tetraaza cyclopenta [fg] acenaphthylène
- 10b, 10c-diméthyl décahydro - 3a,5a,8a, 10a tetraaza pyrène
- décahydro - 3a,5a,8a, 10a tetraaza pyrène

2) hydrolyse, par exemple comme décrit dans l'art antérieur par addition d'une solution aqueuse d'acide chlorydrique, de manière à obtenir le 1,4,7,10-tetraazacyclododécane ou le cyclam ou leurs dérivés substitués sur au moins un carbone du cycle par un groupement aliphatique notamment alkyle ou alkyle-aryle, éventuellement substitués ou interrompus par OH, O, N , CONH, NHCO, -OCO-alkyl, -COO-alkyl
3) le cas échéant alkylation, par des agents alkylants appropriés connus notamment de EP 499 501 ou EP 287 465 (Guerbet), par exemple en utilisant l'acide chloroacétique, l'acide bromoacétique, le bromoacétate de terbutyle et l'acide trifluoracétique, le cas échéant en présence d'une base telle que NaOH ou KOH ou LiOH ;

de manière à obtenir les dérivés DO3A, BT-DO3A, HP-DO3A,DOTA, DOTAM, DOTMA, DOTA-GA le cas échant substitués sur au moins un carbone du cycle polyazoté.

On rappelle que les métaux paramagnétiques incluent les lanthanides de nombre atomique 58-70 et les métaux de transition de nombre atomique 21-29, 42 or 44, par exemple scandium, titane, vanadium, chrome. Avantageusement le métal paramagnétique est choisi parmi les éléments : manganèse, fer, cobalt, nickel, cuivre, molybdène, ruthénium, cérium, praseodymium, neodymium, prométhium, samarium, europium, gadolinium, terbium, dysprosium, holmium, erbium, thulium, et ytterbium.On préfère particulièrement les éléments Gd(III), Mn(II), europium, dysprosium, avantageusement Gd.

Dans le cas d'une utilisation en imagerie multimodale (par exemple IRM + PET) ou en médecine nucléaire (imagerie SPECT et/ou PET), les chélates peuvent être utilisés pour complexer un radioélément tel que le Technecium, l'Indium, le Gallium.

L'invention concerne aussi les compositions décrites précédemment pour leur utilisation pour le diagnostique de maladies, notamment cancéreuses, neurodégénératives, vasculaires.

Selon un autre aspect, le demandeur a réussi à montrer que l'utilisation dans la fabrication des nanoémulsions d'au moins un solvant, avantageusement le chlorophorme et/ou le méthanol, est très avantageuse pour améliorer la stabilisation du nanosystème, la relaxivité des chélates de lanthanides, le temps de demi-vie du produit. L'invention concerne ainsi également la préparation d'une émulsion du demandeur comprenant l'utilisation d'un solvant, avantageusement le chlorophorme et/ou le méthanol. Grâce à l'utilisation de tels solvants, la relaxivité par chélate pour un chélate amphiphile de type q=1 (DOTA, HPDO3A par exemple) passe d'une valeur de 10 à environ 15 $mM^{-1}s^{-1}$.

Selon un autre aspect, l'invention concerne un procédé de préparation d'une nanoémulsion lipidique de lanthanide comprenant une phase lipidique formée de nanogouttelettes lipidiques dispersées en solution aqueuse, comprenant les étapes de :

- préparation d'une phase lipidique comprenant
    - ◦ un premier tensioactif lipide amphiphile
    - ◦ une huile comprenant au moins 70%, de préférence au moins 80, 90, 95, 97 % d'acides gras saturés en C6-C18, avantageusement en C6-C14, et très avantageusement en C6-C10
    - ◦ un chélate de métal paramagnétique amphiphile
- dissolution de la phase lipidique dans un solvant ou un mélange de solvants
- élimination du ou des solvants
- dispersion de la phase lipidique dans une solution aqueuse de manière à former des nanogoutelettes lipidiques
- récupération de la nanoémulsion obtenue.

Avantageusement la phase lipidique est obtenue en mettant ses composants dans un solvant approprié puis en évaporant le solvant.

Les émulsions du demandeur sont des mélanges lipidiques hétérogènes obtenus de manière appropriée, avantageusement par agitation mécanique et/ou addition d'agents émulsifiants. Par exemple, on mélange mécaniquement la phase lipidique représentée par l'huile et les chélates rendus amphiphiles, avec des solvants organiques tels que le chloroforme. Après évaporation du solvant (de manière à former un film lipidique), la phase lipidique est remise en suspension en milieu aqueux (tel que du PBS ou la solution aqueuse de la demande), pour obtenir une émulsion qui subit typiquement une sonication et une microfluidisation. La nanoémulsion obtenue est alors utilisée pour l'administration au patient, le cas échéant après incorporation d'additifs pharmaceutiques variés. Les émulsions obtenues peuvent être lyophilisées avec le cas échéant utilisation d'agents anti agglutination.

La composition formant l'agent de contraste est administrée de préférence en intravasculaire, selon le patient examiné, par exemple à raison de 0.1 mg à 1 g de composé chélate amphiphile et de 1 à 50 micromoles d'ion de métal paramagnétique par kg de patient.

Le demandeur a préparé de nouvelles émulsions, de préférence de lanthanides, ayant des compositions chimiques spécifiques, dont l'efficacité et la tolérance *in vivo* sont significativement améliorées. Les nanoémulsions lipidiques du demandeur sont vectorisées à l'aide de biovecteurs de ciblage (également désignés pharmacophores ou ligands de ciblage). La nanoémulsion comprend au moins un biovecteur de ciblage d'une zone pathologique ancré à la nanoparticule, typiquement à l'aide d'un groupe d'ancrage du biovecteur. Avantageusement, le nombre de biovecteurs par nanoparticule est d'au moins 1000 et typiquement de l'ordre de 1000, 2000, 5000, 10 000.

A titre de ligands de ciblage préférés, on citera des cibles biologiques dont l'expression est modifiée dans une zone pathologique (une tumeur par exemple), par rapport à la zone saine. On utilise avantageusement comme pharmacophore ou ligand de ciblage ou biovecteur de ciblage au moins un ligand choisi parmi : les peptides (avantageusement de moins de 20 acides aminés, plus avantageusement de 5 à 10 acides aminés), les pseudopeptides, les peptidomimétiques, les acides aminés, les agents de ciblage d'intégrines (peptides et pseudopeptides, peptidomimétiques notamment), les glycoprotéines, les lectines, la biotine, les dérivés ptéroïques ou aminoptéroïques, les dérivés de l'acide folique et antifolique, les anticorps ou fragments d'anticorps, l'avidine, les stéroïdes, les oligonucléotides, les séquences d'acide ribonucléique, les séquences d'acide désoxyribonucléique, les hormones, les protéines éventuellement recombinantes ou mutées, les mono- ou polysaccharides, les composés à squelette benzothiazole, benzofurane, styrylbenzoxazole/thiazole/imidazole/quinoline, styrylpiridine et composés dérivés, et leurs mélanges. Les peptides, les dérivés de l'acide folique et antifolique, les agents de ciblage d'intégrines (peptides et pseudopeptides, peptidomimétiques notamment), les agents de ciblage de récepteurs cellulaires ou d'enzymes (notamment de ciblage de kinases, notamment tyrosine kinase ; de métalloprotéases ; de caspases ...) sont particulièrement préférés.

Selon des réalisations avantageuses, le ligand de ciblage est choisi parmi la liste suivante (les documents et références entre parenthèses sont des exemples) :

1) Les biovecteurs ciblant des récepteurs VEGF et angiopoiétine (décrits dans WO 01/97850), les polymères tels que polyhystidine (US 6,372,194), les polypeptides ciblant la fibrine (WO 2001/9188), les peptides de ciblage d'intégrines (WO 01/77145, WO 02 26776 pour alphav beta3, WO 02/081497, par exemple RGDWXE), les pseudopeptides et peptides de ciblage de métalloprotéases MMP (WO 03/062198, WO 01/60416), les peptides ciblant par exemple le récepteur KDR/Flk-1 dont R-X-K-X-H et R-X-K-X-H, ou les récepteurs Tie-1 et 2 (WO 99/40947 par exemple), les glycosides de sialyl Lewis (WO 02/062810 et « Müller et al, Eur. J. Org. Chem, 2002, 3966-3973), les antioxydants tels que l'acide ascorbique (WO 02/40060), les biovecteurs de ciblage de la tuftsine (par exemple US 6,524,554), de ciblage de récepteurs à protéine G GPCR en particulier la cholécystokinine (WO 02/094873), les associations entre antagoniste d'intégrine et mime de la guanidine (US 6 489 333), les quinolones ciblant alphav beta3 ou 5 (US 6,511,648), les benzodiazépines et analogues ciblant des intégrines (US 2002/0106325, WO 01/97861), les imidazoles et analogues (WO 01/98294), les peptides RGD (WO 01/10450), les anticorps ou fragments d'anticorps (FGF, TGFb, GV39, GV97, ELAM, VCAM, inductible par TNF ou IL (US 6,261,535), les molécule de ciblage modifiées par interaction avec la cible (US 5,707,605), les agents de ciblage de dépôts amyloïdes (WO 02/28441 par exemple), les peptides clivés cathepsines (WO 02/056670), les mitoxantrone ou quinone (US 6,410,695), les polypeptides ciblant des cellules épithéliales (US 6,391,280), les inhibiteurs de cystéines protéases (WO 99/54317), les biovecteurs décrits dans : US 6,491,893 (GCSF), US 2002/0128553, WO 02/054088, WO 02/32292, WO 02/38546, WO 03006059, US 6,534,038, WO 01/77102, EP 1 121 377, Pharmacological Reviews (52, n°2, 179 ; facteurs de croissance PDGF, EGF, FGF...), Topics in Current Chemistry (222, W.Krause, Springer), Bioorganic & Medicinal Chemistry (11, 2003, 1319-1341 ; dérivés tétrahydrobenzazépinones ciblant alpha v beta3).

2) Les inhibiteurs d'angiogenèse, notamment ceux testés en essais cliniques ou déjà commercialisés, notamment :

- les inhibiteurs d'angiogenèse impliquant des récepteurs FGFR ou VEGFR tels que SU101, SU5416, SU6668, ZD4190, PTK787, ZK225846, des composés azacycles (WO 02/44156, WO 02/059110) ;
- les inhibiteurs d'angiogenèse impliquant des MMP tels que le BB25-16 (marimastat), le AG3340 (prinomastat), le solimastat, le BAY12-9566, le BMS275291, le metastat, le neovastat ;

- les inhibiteurs d'angiogenèse impliquant des intégrines tels que le SM256, le SG545, des molécules d'adhésion bloquant le EC-ECM (tels que le EMD 121-974, ou la vitaxine) ;
- des médicaments à mécanisme d'action antiangiogenèse plus indirect tels que le carboxiamidotriazole, le TNP470, la squalamine, le ZD0101 ;
- les inhibiteurs décrits dans le document WO 99/40947, les anticorps monoclonaux très sélectifs pour la liaison au récepteur KDR, les analogues de la somatostatine (WO 94/00489), les peptides de liaison à la sélectine (WO 94/05269), des facteurs de croissance (VEGF, EGF, PDGF, TNF, MCSF, interleukines); des biovecteurs de ciblage de VEGF décrits dans Nuclear Medicine Communications, 1999, 20 ;
- les peptides inhibiteurs du document WO 02/066512.

3) Des biovecteurs capables de cibler des récepteurs : CD36, EPAS-1, ARNT, NHE3, Tie-1, 1/KDR, Flt-1, Tek, neuropiline-1, endogline, pléientropine, endosialine, Axl., alPi, a2ssl, a4P1, a5pl, eph B4 (éphrine), récepteur laminine A, récepteur neutrophiline 65, récepteur leptine OB-RP, récepteur chimiokine CXCR-4 (et autres récepteurs cités dans le document WO99/40947), LHRH, bombésine/GRP, récepteurs gastrine, VIP, CCK.

4) Des biovecteurs de type inhibiteurs de tyrosine kinase.

5) Les inhibiteurs du récepteur GPIIb/IIIa connus choisis parmi : (1) le fragment fab d'un anticorps monoclonal du récepteur GPIIb/IIIa, Abciximab, (2) les petites molécules peptidiques et peptidomimétiques injectées en intraveineuse telles que l'eptifibatide et le tirofiban.

6) Des peptides antagonistes de récepteurs au fibrinogène (EP 0 425 212), des peptides ligands de récepteurs IIb/IIIa, des ligands du fibrinogène, des ligands de la thrombine, des peptides capables de cibler la plaque d'athérome, les plaquettes, la fibrine, des peptides à base d'hirudine, des dérivés à base de guanine ciblant le récepteur IIb/IIIa.

7) D'autres biovecteurs ou fragments biologiquement actifs de biovecteurs connus de l'homme du métier comme médicaments, à action anti-thrombotique, anti agrégation plaquettaire, antiathérosclérotique, antiresténotique, anticoagulante.

8) D'autres biovecteurs ou fragments biologiquement actifs de biovecteurs ciblant alpha v beta3, décrits en association avec des DOTA dans le brevet US 6 537 520, choisis parmi les suivants : mitomycine, tretinoine, ribomustine, gemcitabine, vincristine, etoposide, cladribine, mitobronitol, methotrexate, doxorubicine, carboquone, pentostatine, nitracrine, zinostatine, cetrorelix, letrozole, raltitrexed, daunorubicine, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoine, streptozocine, nimustine, vindesine, flutamide, drogenil, butocin, carmofur, razoxane, sizofilan, carboplatine, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, leutinizing hormone releasing factor.

9) certains biovecteurs ciblant des types particuliers de cancers, par exemple des peptides ciblant le récepteur ST associé au cancer colorectal, ou le récepteur tachykinine.

10) des biovecteurs utilisant des composés de type phosphines.

11) des biovecteurs de ciblage de P-sélectine, E-sélectine ; par exemple, le peptide de 8 acides aminés décrit par Morikawa et al, 1996, 951, ainsi que différents sucres.

12) l'annexine V ou des biovecteurs ciblant les processus apoptotiques.

13) tout peptide obtenu par des technologies de ciblage telles que le phage display, modifié éventuellement par des acides aminés non naturels (http//chemlibrary.bri.nrc.ca), par exemple des peptides issus de banques phage display : RGD, NGR, CRRETAWAC, KGD, RGD-4C, XXXY*XXX, RPLPP, APPLPPR.

14) d'autres biovecteurs peptidiques connus de ciblage de plaques d'athérome, cités notamment dans le document WO 2003/014145 et notamment VCAM

15) des vitamines.

16) des ligands de récepteurs hormonaux dont les hormones et les stéroïdes.

17) des biovecteurs ciblant des récepteurs opioïdes.

18) des biovecteurs ciblant des récepteurs TKI.

19) des antagonistes LB4 et VnR.

20) des composés nitriimidazoles et benzylguanidines.

21) des biovecteurs rappelés dans Topics in Current Chemistry, vol.222, 260-274, Fundamentals of Receptor-based Diagnostic Metallopharmaceuticals, notamment :

- des biovecteurs de ciblage de récepteurs peptidiques surexprimés dans les tumeurs (récepteurs LHRH, bombésine/GRP, récepteurs VIP, récepteurs CCK, récepteurs tachykinine par exemple), notamment les analogues de somatostatine ou de bombésine, des peptides dérivés octréotide éventuellement glycosylés, les peptides VIP, les alpha-MSH, les peptides CCK-B

- des peptides choisis parmi : des peptides cycliques RGD, fibrine-chaîne alpha, CSVTCR, tuftsine, fMLF, YIGSR (récepteur : laminine).

22) des oligosaccharides, des polysaccharides et des dérivés d'oses, des dérivés ciblant les récepteurs Glut (récepteurs d'osés).
23) des biovecteurs utilisés pour des produits de type smart.
24) des marqueurs de la viabilité myocardique (tétrofosmine et hexakis2méthoxy-2méthylpropylisonitrile).
25) des traceurs du métabolisme des sucres et des graisses.
26) des ligands de récepteurs de neurotransmetteurs (récepteurs D, 5HT, Ach, GABA, NA).
27) des oligonucléotides.
28) le facteur tissulaire
29) des biovecteurs décrits dans WO 03/20701, en particulier le PK11195 ligand du récepteur périphérique aux benzodiazépines.
30) des peptides liant la fibrine, notamment les séquences peptidiques décrites dans WO 03/11115.
31) des inhibiteurs d'agrégation de plaques amyloïdes (décrits par exemple dans WO 02/085903).
32) des pharmacophores composés de ciblage de la maladie d'Alzheimer, en particulier les composés comprenant les squelettes de type benzothiazole, benzofuranes, styrylbenzoxazoles/thiazoles/imidazoles/quinoline, styrylpiridines.
33) des pharmacophores composés de ciblage obtenus à partir de squelettes chimiques à activité pharmacologique décrits dans US2007098631, notamment les formules des pages 4 à 10 et pages 13 -14 (incorporé par référence), notamment les composés du tableau page 4 dans la colonne intitulée « scaffolds and derivatives » : biphenyl; arylpiperidine; arylpiperazine; 1,4 dihydropyridine dihydropyrimidone; 1,4 benzodiazepine-2-one; 1,5 benzodiazepine-2-one; 1,4-benzodiazepine-2,5 diones; pyrrolo2,1-c 1,4 benzodiazepines 5,11 diones; 1,4 benzothiazepine-5-ones; 5,11-dihydro-benzo pyrido 3,2b 1,4 diazepine-6-ones benzopyrane; chromone; benzopyranone; coumarine, pyranocoumarine; benzopiperazinones; quinazolinone; quinazolindione; quinoxalinone; imidazoquinoxaline; indole; benzimidazole, benzofurane, benzothiophene.
34) des composés de ciblage d'intégrines notamment ayant une affinité supérieure à 10 000, 100 000 ou plus, notamment des composés non peptidiques mimétiques de peptides RGD, et en particulier des composés tetrahydro naphtyridine décrits par exemple dans : J Med. Chem., 2003, 46, 4790-4798, Bioorg. Med. Chem. Letters, 2004, 14, 4515-4518, Bioorg. Med. Chem. Letters, 2005, 15, 1647-1650.
35) des composés de ciblage de MUC5AC, notamment des fragments d'anticorps, des peptides et des composés non peptidiques mimétiques de peptides

En particulier pour ces composés naphtyridine, le demandeur utilise tout composé de naphtyridine connu de l'art antérieur (notamment ceux de WO 2009/114776), l'utilisation des composés naphtyridine comme biovecteur pour imagerie médicale étant décrite dans WO 2007/042506 page 13 lignes 30-34.

Les ligands de ciblage (partie biovecteur du biovecteur amphiphile) pour la reconnaissance de la cible en milieu biologique sont greffés, essentiellement du côté de la surface externe des nanogouttelettes, à l'aide de groupes chimiques d'ancrage dans la couche de tensioactif appropriés.

Le biovecteur amphiphile s'écrit avantageusement sous la forme : Bio - L - Lipo dans laquelle :

- Bio est la partie de reconnaissance biologique localisée à la surface externe des nanogouttelettes
- Lipo est un groupe lipophile permettant l'insertion du biovecteur au sein de la couche de tensioactif
- L est un groupe de liaison reliant Bio et Lipo, avantageusement choisi parmi :

  - rien ou une simple liaison, C1-6 alkylène, PEG par exemple CH2-(CH2-O-CH2)k-CH2, $(CH_2)_3$-NH, NH-$(CH_2)_2$-NH, NH-$(CH_2)_3$-NH, $(CH_2)_n$, $(CH_2)_n$-CO-, -$(CH_2)_n$NH-CO- avec n = 2 à 10 , $(CH_2CH_2O)_q(CH_2)_r$-CO- , $(CH_2CH_2O)$q$(CH_2)_r$-NH-CO- avec q = 1-10 et r=2-10, $(CH_2)_n$-CONH - , $(CH_2)_n$-CONH - PEG, $(CH_2)_n$-NH- HOOC-$CH_2$-O-$(CH_2)_2$-O-$(CH_2)_2$-O-$CH_2$-COOH ; HOOC-$(CH_2)_2$-$CO_2$-$(CH_2)_2$-OCO-$(CH_2)_2$-COOH ; HOOC-CH(OH)-CH(OH)-COOH; HOOC-$(CH_2)_n$-COOH; $NH_2$-$(CH_2)_n$-$NH_2$, avec n=0-20; $NH_2$-$(CH_2)_n$-$CO_2$H ; $NH_2$-$CH_2$ - $(CH_2$-O-$CH_2)_n$-$CO_2$H avec n=1 à 10, squarate
  - P1-1-P2, identiques ou différents, P1 et P2 étant choisis parmi O, S, NH, rien, $CO_2$, NHCO, CONH, NHCONH, NHCSNH, $SO_2$NH-, $NHSO_2$-, squarate

avec 1 = alkyle, alkoxyalkyle, polyalkoxyalkyle (PEG), alkyle interrompu par un ou plusieurs squarates ou par un ou plusieurs aryles, avantageusement phényles, alcényle, alcynyle, alkyle interrompus par un ou plusieurs groupes choisis parmi -NH-, -O-, -CO-, -NH(CO)-, -(CO)NH-, -O(CO)-, ou - (OC)O-).

Les liaisons covalentes entre Bio et L sont avantageusement de type -CONH-, -COO-, - NHCO-, -OCO-, -NH-CS-NH-, -C-S-, -N-NH-CO-, -CO- NH-N-, -CH2-NH-, -N-CH2-, -N-CS-N-, -CO-CH2-S-, -N-CO-CH2-S-, -N-CO-CH2-CH2-S-, -CH=NH-NH-, -NH-NH=CH-, - CH=N-O-, -O-N=CH- ou répondant aux formules suivantes :

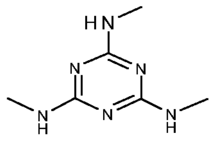
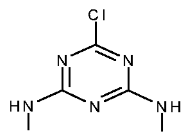
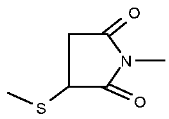
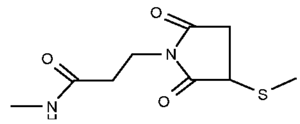

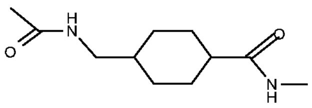
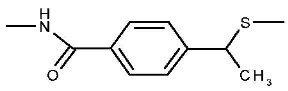
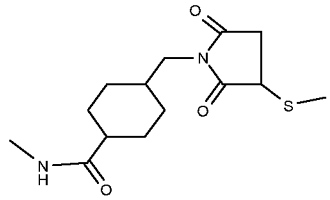

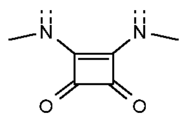

On présente quelques exemples de biovecteurs pharmacophores, petites molécules organiques de ciblage (ci après : peptides, dérivés de l'acide folique, dérivés naphtyridine), rendus amphiphiles pour l'ancrage à la surface externe de la nanoparticule .

La demande présente des exemples illustratifs de leur synthèse.

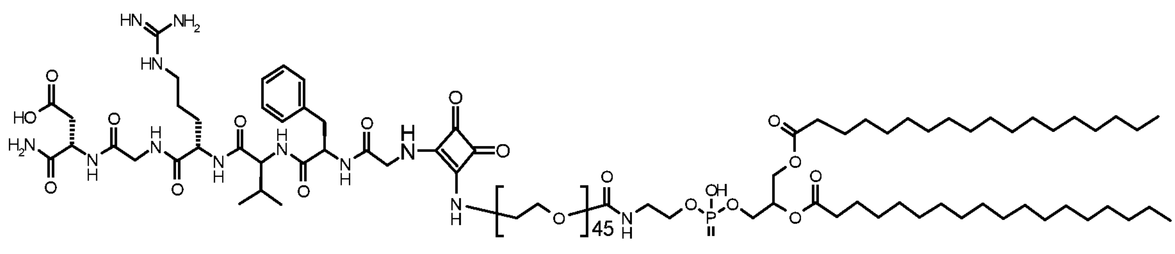

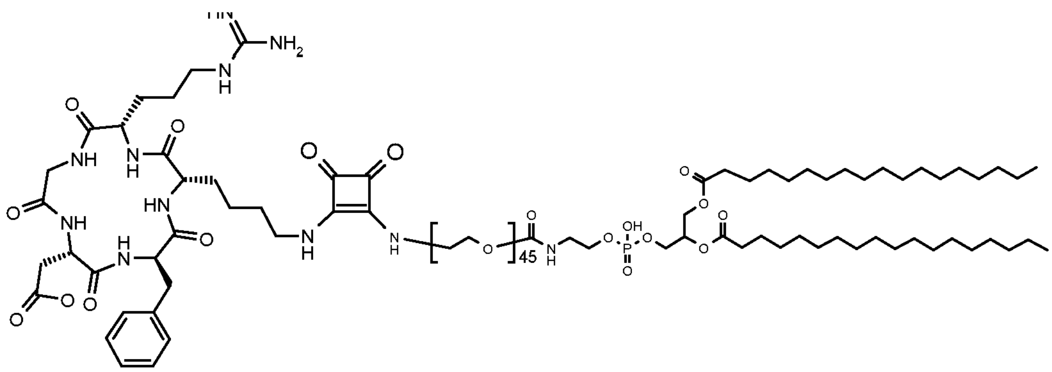

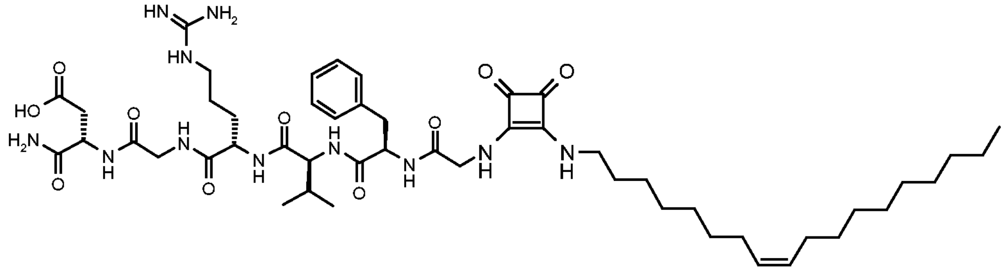

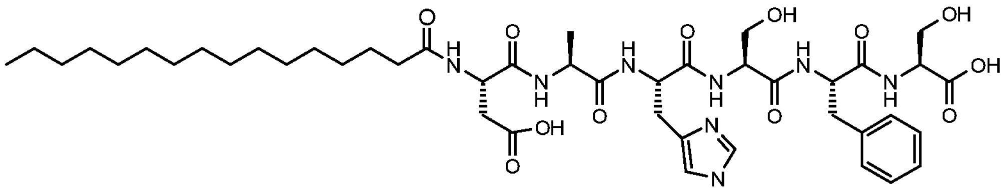

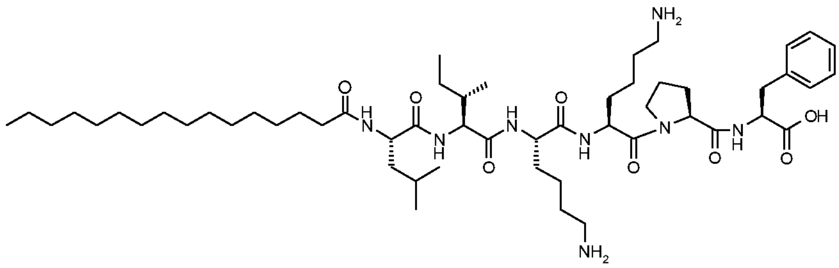

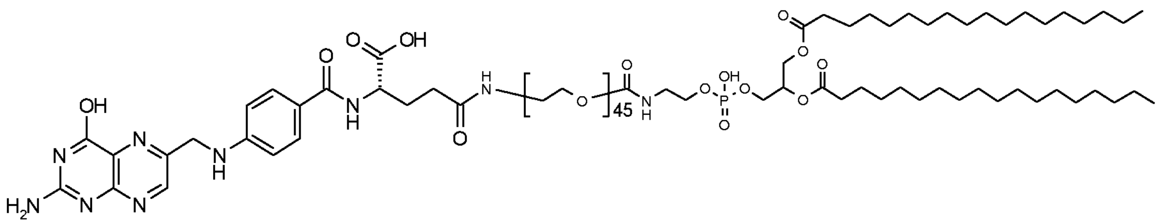

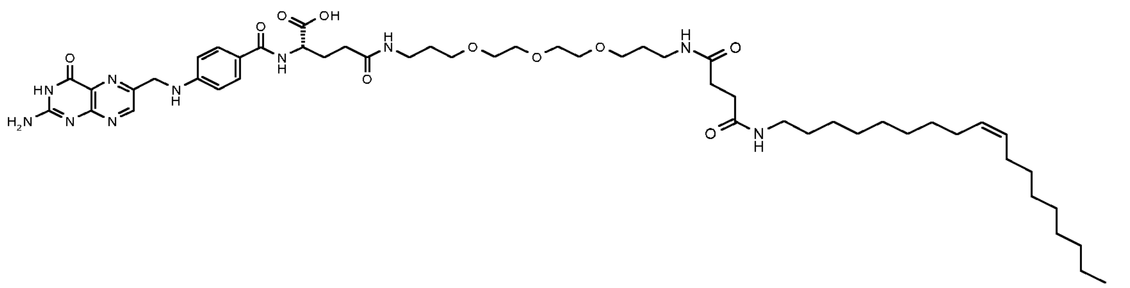

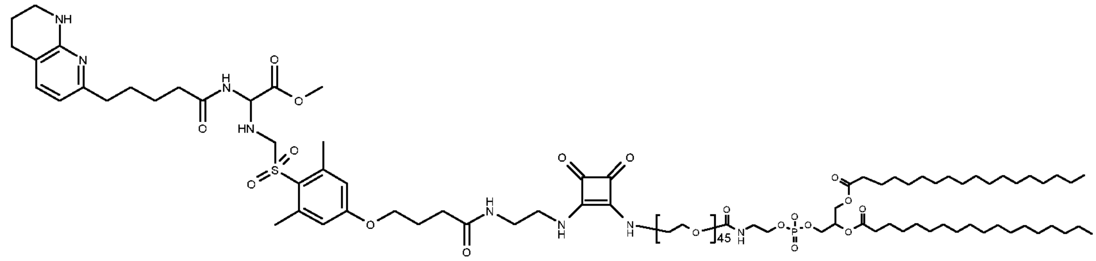

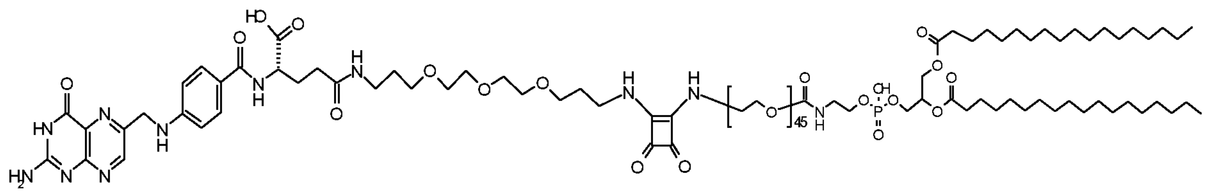

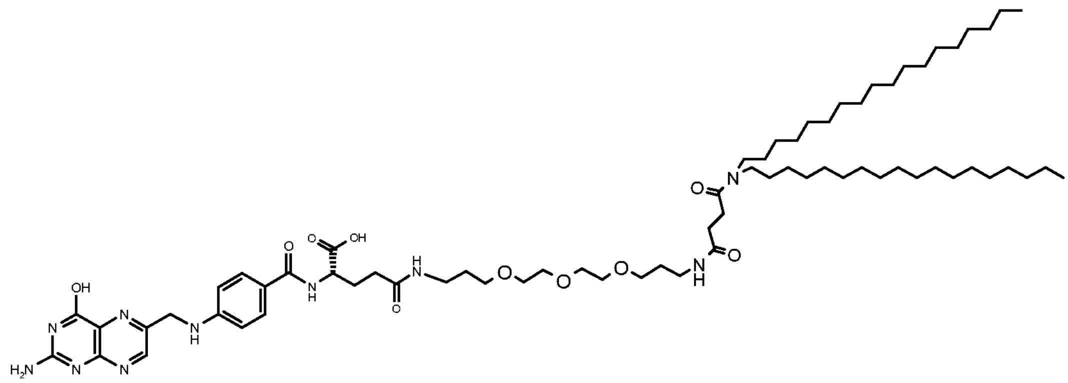

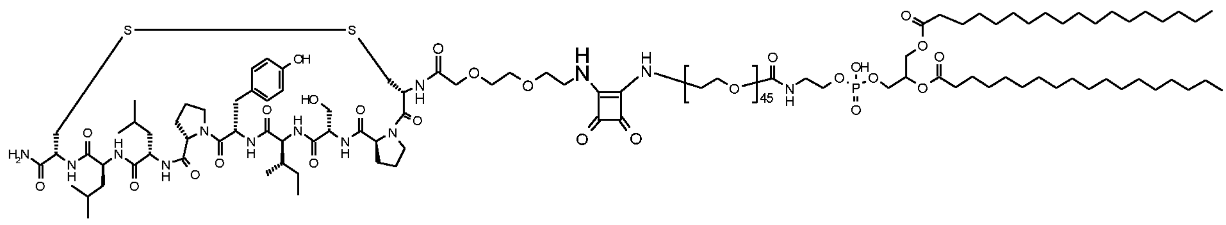

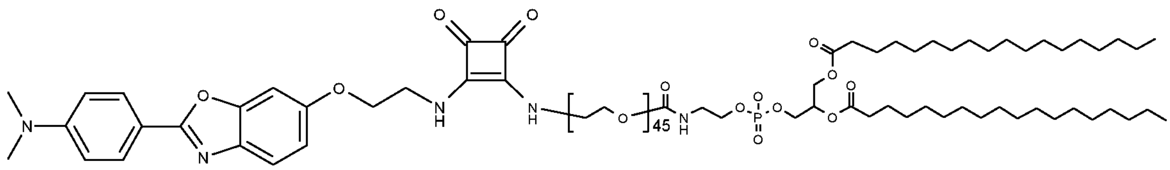

Comme expliqué dans la demande, les ligands de ciblage cités sont essentiellement à vocation d'imagerie diagnostique. On peut toutefois préparer des nanoémulsions comprenant en outre des ligands également à but de traitement thérapeutique. Les nanogouttelettes comprendront alors d'une part un ligand de ciblage pour atteindre la cible biologique (la zone pathologique), et d'autre part un ligand utilisé comme médicament pour le traitement thérapeutique. L'invention concerne ainsi aussi les compositions décrites précédemment, lorsqu'elles incorporent un pharmacophore thérapeutique, pour leur utilisation pour le traitement de maladies, notamment cancéreuses, neurodégénératives, vasculaires.

Selon des réalisations, le tensioactif comprend en outre au moins un agent de furtivité amphiphile, avantageusement un dérivé PEG, un dérivé de type ganglioside (résidus osidiques estérifiés typiquement par l'acide sialique ou le NAC), un polysaccharide (notamment le dextran ou l'un de ses dérivés connus). Ces agents de furtivité sont intégrés sans altérer l'affinité de la nanoparticule pour la cible biologique.

La composition formant l'agent de contraste est administrée de préférence en intravasculaire, selon le patient examiné, par exemple à raison de 0,1 mg à 1 g de composé chélate amphiphile et de 1 à 50 micromoles d'ion de métal paramagnétique par kg de patient.

Les compositions lipidiques obtenues sont le cas échéant formulées à l'aide d'additifs connus rappelés par exemple dans US 6,010,682, notamment pour une administration par injection intraveineuse. On citera notamment des agents épaississants, des saccharides ou polysaccharides, le glycérol, le dextrose, le chlorure de sodium, des antimicrobiens.

Avantageusement, grâce aux compositions selon l'invention, on peut obtenir une augmentation de la relaxivité par ion. On obtient typiquement les caractéristiques suivantes, pouvant varier selon les compositions précises des émulsions et leur procédé de préparation :

- index de polydispersité : 0,2 à 0,3
- $[Gd^{3+}]$ = 2 à 10 mM de préférence 3 à 7 mM
- concentration en particules : 50 à 100 nM
- r1(mM $^{-1}$S $^{-1}$Gd$^{-1}$) : 5 à 40, de préférence 10 à 40
- r2(mM $^{-1}$S $^{-1}$Gd$^{-1}$) : 20 à 40
- r1(mM $^{-1}$S$^{-1}$ particule-1) : $10^6$ à $4x10^6$
- nombre de biovecteurs : 50 à 10000, notamment 1000 à 5000, avantageusement 1500 à 2500.

Au global, le demandeur a réussi à obtenir de nouvelles nanoémulsions pour l'IRM

- suffisamment stables chimiquement pour être produites et conservées une longue durée (plusieurs mois à plusieurs années), en particulier sans problème de coalescence entre elles des gouttelettes lipidiques
- suffisamment stables *in vivo* pour ne pas être dégradées
- adaptées sur le plan de la pharmacocinétique
- suffisamment efficaces en terme de signal pour l'imagerie clinique (IRM en particulier) chez le patient
- capables d'incorporer des ligands de ciblage de zones pathologiques à la surface des nanogouttelettes, en quantité appropriée et sans perte d'affinité gênante avec leur cible biologique.

L'invention concerne aussi :

- un produit de contraste, de préférence pour IRM, comprenant les formulations des nanoémulsions de la demande
- les nanoémulsions du demandeur pour leur utilisation dans le diagnostique notamment de maladies cancéreuses, inflammatoires, neurodégénératives, cardiovasculaires.

Le demandeur a par ailleurs étudié des procédés destinés à récupérer des lanthanides, pour des raisons de coût et de préservation de l'environnement, lors de la synthèse d'agents de contraste et notamment d'émulsions de lanthanides, comme cela se fait pour des produits de contraste iodés. Par exemple, pour les produits de contraste iodés, des procédés de récupération d'iode existent, soit par oxydation catalytique (en présence de cuivre notamment et à une température par exemple de 150°C), soit par oxydation thermique avec combustion des effluents de fabrication des produits iodés. Par exemple, l'oxydation thermique est réalisée par incinération, les effluents, préalablement mélangés ou non mélangés à une solution de métal alcalin, sont injectés au contact de la flamme à plus de 800°C d'un incinérateur, une solution de métal alcalin pouvant de manière optionnelle également être injectée au contact de flamme, les fumées issues de l'incinération desdits effluents sont ensuite absorbées/passées dans une solution aqueuse avantageusement de soude par exemple à environ 70-90°C ; après éventuel refroidissement, la solution obtenue contenant typiquement des ions iodures et éventuellement chlorures et sulfates, est additionnée d'un agent oxydant, eau oxygénée par exemple, en milieu acide par exemple à pH inférieur à 1 ou 2 et à une température de préférence de 10 à 40°C notamment autour de 20°C. L'iode en solution obtenue après oxydation est ensuite récupérée par différents types de traitements possibles, par exemple un traitement mécanique (décantation, absorption sur supports, précipitation, filtration ...) ou un traitement chimique de conversion en composés iodés valorisables. De manière analogue, des procédés de récupération sont intéressants pour récupérer du gadolinium à partir des effluents gadolinés issus de la synthèse des produits de contraste gadolinés, utilisant par exemple des traitements acides adaptés et/ou des résines dédiées.
L'invention est illustrée à l'aide des exemples qui suivent.

**Exemple 1 :** synthèse d'un dérivé DTPA lipophile

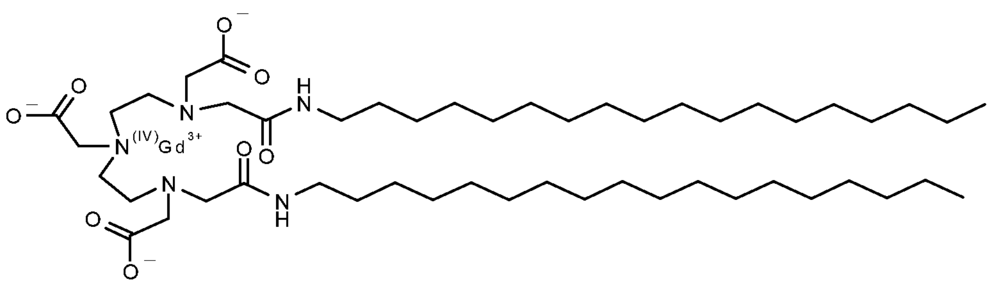

Etape 1 :

6g de DTPA bis anhydride sont mis en suspension dans 240ml de DMF. La suspension est chauffée à 50°C et il y a passage en solution. L'octadécylamine est ajouté en une seule fois. La réaction est maintenue 1 nuit à 50°C. Le milieu réactionnel est refroidi puis filtré sur fritté. Le précipité est lavé une fois au DMF puis abondamment au méthanol. 13.5 g de poudre jaune-blanche sont obtenus avec un rendement (Rdt) de 90%. L'analyse de spectrométrie de masse se fait par infusion de l'échantillon en ES+.
$C_{50}H_{97}N_5O_8$ ; m/z (ES+) = 896

Etape 2 :

13.4g de ligand (Int3) sont mis en suspension dans 600ml de méthanol. 6.67g de $GdCl_3,6H_2O$ sont ajoutés. Il y a solubilisation instantanément. Le pH de la solution est ajusté à 7 à l'aide d'une solution de méthanolate de sodium dans le méthanol (2.68g de $CH_3ONa$ dans 400ml de $CH_3OH$). La solution est portée au reflux pendant 45 min. Le méthanol est évaporé et le résidu repris à l'eau. La poudre est lavée abondamment à l'eau. 15 g de produit brut sont obtenus avec un rendement de 96%. Le produit est purifié par chromatographie flash sur gel de silice. On purifie 15 g avec une phase éluante composée de 90/10 en méthanol-dichlorométhane. Après purification, 10 g de produit pur sont obtenus (poudre blanche grasse).
$C_{50}H_{94}GdN_5O_8$ ; m/z (ES-) = 1049

**Exemple 2 :** Synthèse de dérivés PCTA lipophiles

**Exemple 2.1 :** DSPE - PCTA

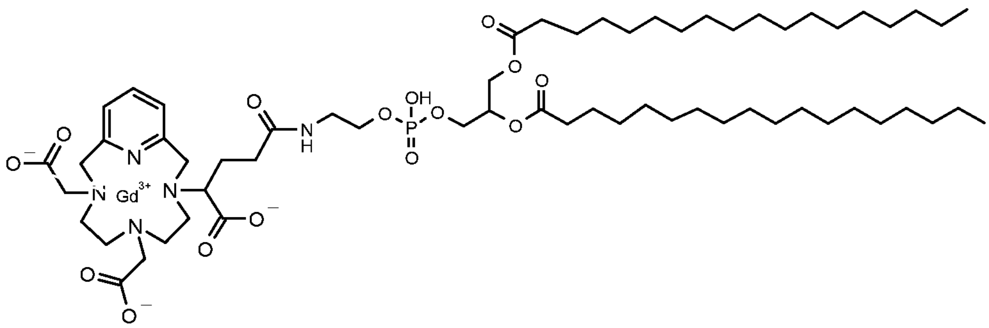

Etape 1 :

576 mg de l'Int 1 sont mis en suspension dans 3mL de DMSO. Les agents activants sont introduits, 1,1 eq d'EDCI soit 219 mg et 1,1 eq de NHS soit 131mg. Au bout d'une nuit, il y a passage en solution (l'ester s'est formé).
$C_{24}H_{28}GdN_5O_{10}$ ; m/z (ES-) = 703

Etape 2 :

La DSPE (leq, 711 mg) sont dissous dans un minimum de pyridine à 90°C. Une fois solubilisée, la solution est coulée lentement sur la solution de DMSO contenant l'ester activé ; la réaction est laissée 10 min à 90°C puis on laisse réagir en laissant tomber la température pendant 1 nuit. Le milieu réactionnel est précipité dans l'eau froide et est centrifugé. Le culot est lavé à l'eau puis à nouveau centrifugé. Le culot est repris au méthanol puis évaporé à sec. On obtient environ 400mg de produit brut qui est ensuite purifié par chromatographie flash sur gel de silice (cartouches de 30g).

On utilise dans un premier temps une phase éluante constituée de DCM-MeOH 88-12 avec acide formique pour éliminer la DSPE résiduelle puis on décroche le produit attendu avec le mélange quaternaire DCM/MeOH/eau/acide formique 65/25/4/1. $C_{61}H_{105}GdN_5O_{15}P$ ; m/z (ES-) = 1335

**Exemple 2.2 :** autres exemples de chélates PCTA lipophiles

Exemple 19 de WO 2006/100305

b) Complexe de gadolinium de l'ester 3-[(2-{3,4-dioxo-2-[3-(3,6,9-tris-carboxyméthyl-3,6, 9,15-tétraaza-bicyclo[9.3.1]pentadéca-1(14),11(15),12-trièn-13-yl)-propylamino]-cyclobut-1-énylamino}-éthoxy)-hydroxy-phosphoryloxy]-2-octadécanoyloxy-propylique de l'acide Octadécanoique

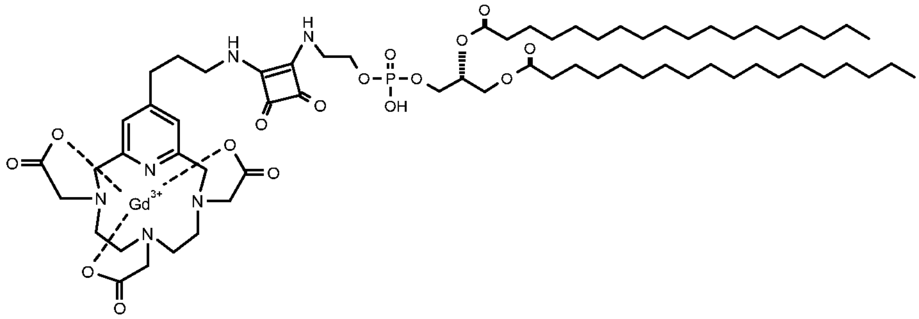

Selon le mode opératoire de l'étape a) de l'exemple 18 de WO 2006/100305, au départ de 500mg du composé préparé à l'étape a) de l'exemple 12 de WO 2006/100305 et 520mg de DSPE. m= 350 mg
m/z: ES- 1417

Exemple 20 de WO 2006/100305

a) Complexe de gadolinium de l'acide 2-(3,9-Bis-carboxymethyl-3,6,9,15-tetraaza-bicyclo[9.3.1]pentadeca-1(14),11(15),12-trien-6-yl)-5-hexadécanoylamino-pentanoique

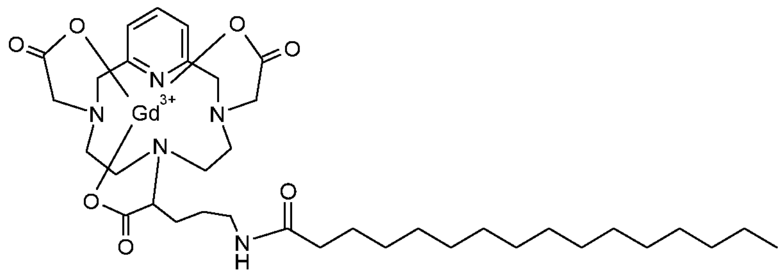

Selon le mode opératoire de l'étape a) de l'exemple 6 de WO 2006/100305 au départ du composé obtenu à l'étape c) de l'exemple 13 de WO 2006/100305 (300mg) et 150mg du chlorure de l'acide palmitique.m= 230mg m /z : ES- 829

Exemple 21 de WO 2006/100305

a) Complexe de gadolinium de l'ester 3-({2-[5-(3,9-bis-carboxyméthyl-3,6,9,15-tétraaza-bicyclo[9.3.1]pentadéca-1(14),11(15), 12-trièn-6-yl)-5-carboxy-pentanoylamino]-éthoxy}-hydroxy-phosphoryloxy)-2-héxadecanoyloxy-propylique de l'acide Hexadécanoique

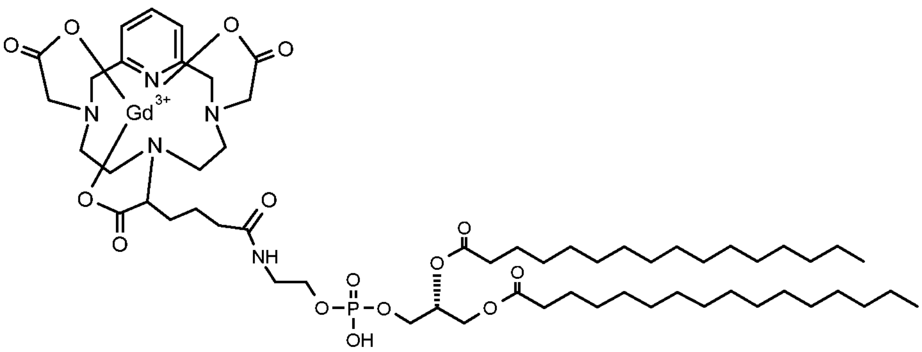

Selon le mode opératoire de l'étape a) de l'exemple 8 de WO 2006/100305, au départ de 100mg du composé préparé à l'étape d) de l'exemple 15 de WO 2006/100305 et 120mg de DPPE. m= 80 mg
m /z : ES- 1293

Exemple 5 de WO 2006/100305

a) Complexe de gadolinium de l'ester de l'acide Octadécanoique 3-({2-[4-(3,9-bis-carboxyméthyl-3,6,9,15-tétraaza-bicyclo[9.3.1]pentadéca-1(14),11(15), 12-trien-6-yl)-4-carboxy-butyrylamino]-éthoxy}-hydroxy-phosphoryloxy)- 2-octa-décanoyloxy-propylique

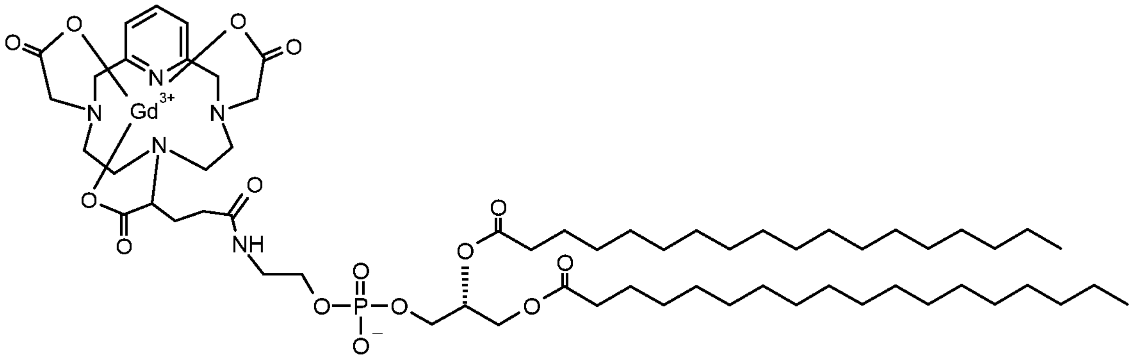

200 mg du composé obtenu à l'étape c) de l'exemple 3 de WO 2006/100305 sont dissous dans 10 ml de Diméthylformamide. A cette solution sont ajoutés 204 mg de N,N'-dicyclohexylcarbodiimide et 40mg de N-hydroxysuccinimide. Le mélange est agité 1h à température ambiante et une solution de 250 mg de 1,2-Distearoyl-sn-Glycero-3-Phosphoethanolamine (DSPE, AVANTI® Polar Lipids, Inc.) dans 5 ml de pyridine est ajoutée. Le milieu réactionnel est agité 20h à température ambiante puis précipité dans 50 ml d'éthanol .Le produit est ensuite purifié sur gel de silice. m= 190mg.
m/z : ES- 1335

Exemple 6 de WO 2006/100305

a) Complexe de gadolinium de l'acide 2-(3,9-Bis-carboxyméthyl-3,6,9,15-tétraaza-bicyclo[9.3.1]pentadéca-1(14),11(15),12-trien-6-yl)-4-(4-octadéc-9-ènoylamino-phényl)-butyrique

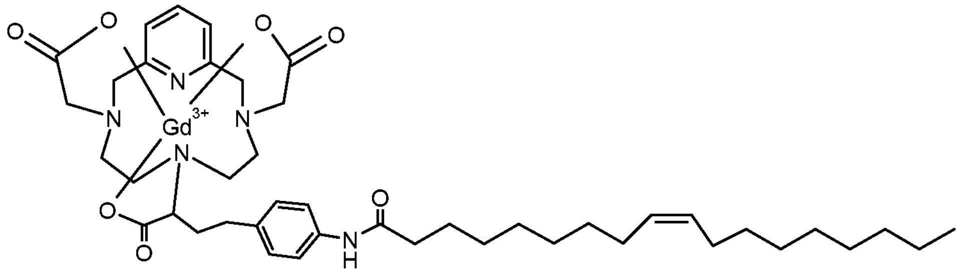

500mg du composé obtenu à l'étape j) de l'exemple 1 de WO 2006/100305 sont dissous dans 30 ml de DMSO anhydre. 230 mg de triéthylamine sont ajoutés puis 400mg de chlorure de l'acide Oléique (ALDRICH®). Le mélange est agité 6h à température ambiante et précipité dans l'éthanol. Le produit est ensuite purifié sur gel de silice.m=300 mg. m/z : ES- 917

Exemple 8 de WO 2006/100305

a) Complexe de gadolinium de l'ester 2-héxadécanoyloxy-3-(hydroxy-{2-[2-(3,6,9-tris-carboxyméthyl-3,6,9,15-tétraaza-bicyclo[9.3.1]pentadéca-1(15),11,13-trien-12-yloxy)-acétylamino]-éthoxy}-phosphoryloxy)-propylique de l'acide Hexa-décanoique

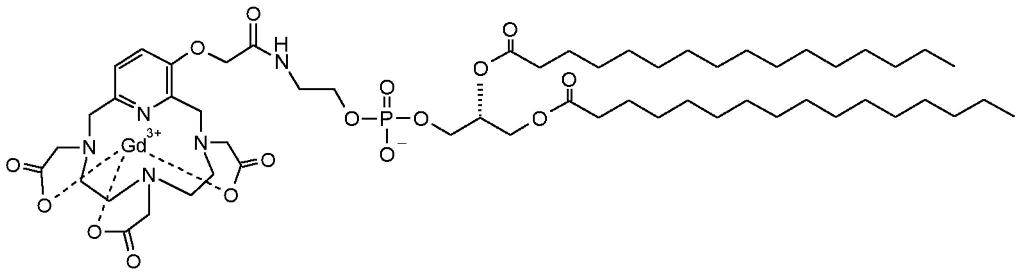

**Exemple 3 :** Synthèse de dérivés DOTA lipophiles

**Exemple 3.1 :** DOTA-DSPE

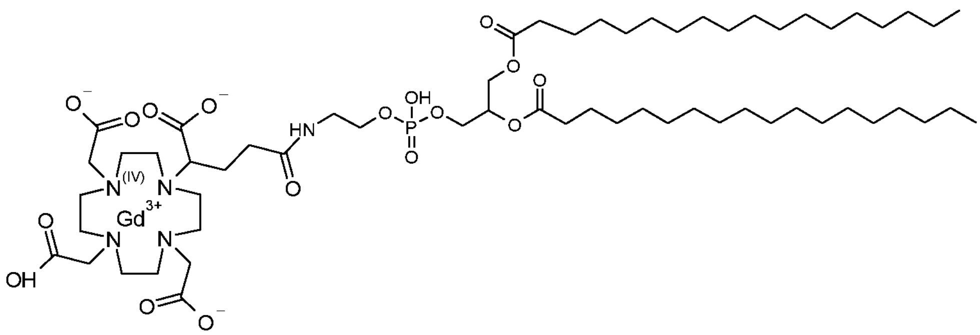

200 mg de DOTA-Gd carboxylate sont mis en solution dans 1.5 ml d'eau. 238 mg de 1,2-dioctadecanoyl-*sn*-glycero-3-phosphoethanolamine (DSPE) solubilisés dans 70 ml de pyridine à 80°C sont ajoutés ainsi que 85 mg de EDCI et 22mg d'HOBT. Le milieu réactionnel est agité à 40°C pendant 24h. La pyridine est ensuite évaporée et le résidu est repris dans l'éthanol puis filtré.
$C_{60}H_{108}GdN_5O_{17}P$ ; m/z ($ES^-$) = 1358

**Exemple 3.2**

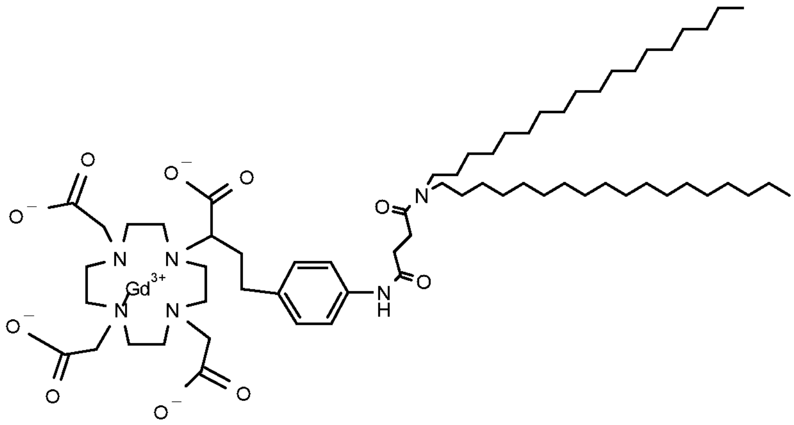

Etape 1 :

1.043 g de N-dioctadecylamine et 200 mg d'anhydride succinique sont dissous dans 10 ml de pyridine. Après 2h à 50°C, le milieu réactionnel est précipité dans 100ml d'eau acidifiée ; filtré et lavé à l'eau acidifiée. Après séchage sous vide, 1.12g d'une poudre blanche sont obtenus. $C_{40}H_{79}NO_3$ ; m/z ($ES^-$) = 621

Etape 2 :

L'ester activé de NHS est obtenu en faisant réagir 300mg de composé obtenu à l'étape 1 dans 5 ml de dichlorométhane avec 100 mg de Dicyclohexylcarbodiimide et 56 mg de N-Hydroxysuccinimide. Après 30 minutes, le précipité formé est filtré. Le filtrat est engagé dans l'étape suivante sans concrétisation.
$C_{44}H_{82}N_2O_5$ ; m/z ($ES^-$) = 718

Etape 3 :

Le filtrat obtenu à l'étape 2 est ajouté goutte à goutte à 360mg de complexe de gadolinium dissous dans 3 ml de DMSO et 20 $\mu$l de triéthylamine. Le milieu réactionnel est agité à 3heures à température ambiante. Après évaporation du dichlorométhane, le milieu réactionnel est précipité dans l'eau puis filtré. Le précipité est ensuite purifié sur silice normale avec une élution par mélange dicholromethane/méthanol. 30mg de produit sont obtenus.
$C_{64}H_{111}GdN_6O_{10}$ ; m/z ($ES^-$) = 1280,8

**Exemple 3.3 :**

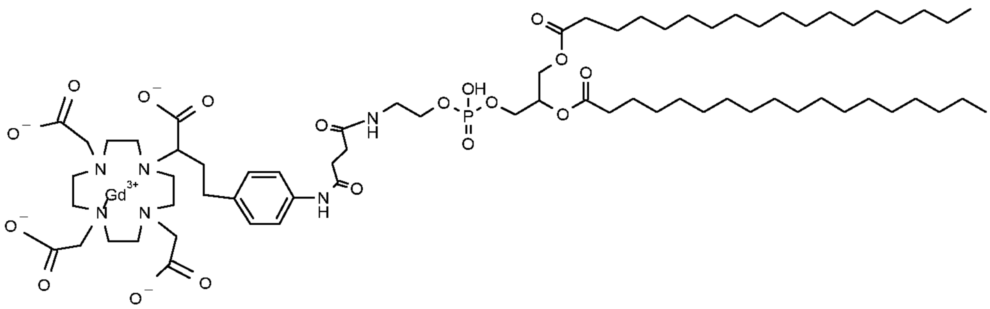

Etape 1 :

La réaction est menée dans les mêmes conditions que l'étape 1 de l'exemple 3.2, la N-dioctadecylamine étant

remplacé par 1.495g de DSPE.
$C_{45}H_{86}NO_{11}P$ ; m/z (ES-) = 846.6

Etape 2 :

Identique à l'étape 2 de l'exemple 3.2 ; à partir du composé obtenu à l'étape 1 , 41mg de NHS et 73mg de DCC.
$C_{49}H_{89}N_2O_{13}P$ ; m/z ($ES^+$) = 944

Etape 3 :

Identique à l'étape 2 de l'exemple 3.2.
$C_{69}H_{118}GdN_6O_{18}P$ ; m/z ($ES^-$) = 1508

**Exemple 3.4 :** Exemple 33 de WO 2010/066815

e) Synthèse du (4, 7-bis-carboxymethyl-10-doctadecylcarbamoylmethyl-1,4,7,10tetraaza-cyclodode-1-yl)-acetic acid

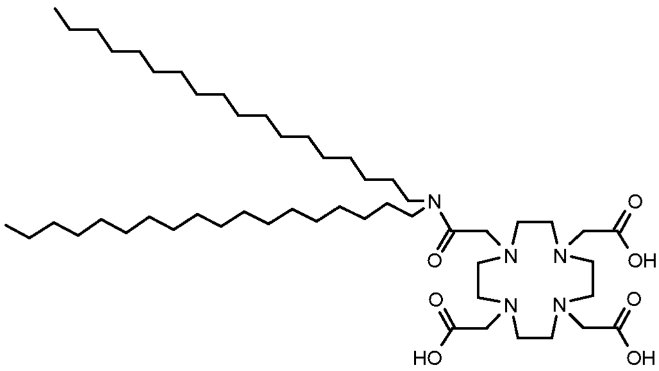

Dans un ballon de 25mL, 40mg de l'intermédiaire obtenu en d) de WO 2010/066815 (0,037mmol ; léquiv) sont dissous dans 4mL d'acide trifluoroacétique. et 1mL de $CH_2Cl_2$ pendant 5h. Après évaporation du mélange de solvants, le solide jaune (32mg) est précipité dans l'éther diéthylique puis filtré sur fritté.
$C_{52}H_{101}N_5O_7$ ; MALDI-TOF mode positif m/z 908,76

Les exemples suivants illustrent la synthèse de ligands de ciblage amphiphiles, avec utilisation de certains linkers, l'homme du métier sait adapter les protocoles pour d'autres linkers par exemple alkyle C1-C10, PEG, alkylène C1-C10 - PEG - alkylène C1-C10, squarate, alkylène-PEG-alkylène.

**Exemple 4 :** synthèse d'un peptide RGD lipophile (peptide RGD linéaire)

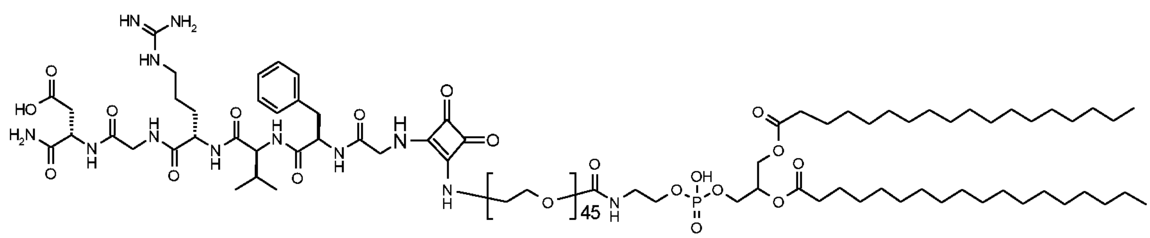

Etape 1

100mg (0,15mmol) de peptide H-Gly-(D)-Phe-(L)-Val-(L)-Arg-Gly-(L)Asp-$NH_2$ (H-GfVRGD-NH2) acheté chez Bachem sont dissous sous argon dans 3ml de DMSO séché sur tamis. 23μl de 3,4-Diethoxy-3-cyclobutene-1,2-dione (0.15mmol ; léquiv) et 25μl de triéthylamine sont ajoutés. Le milieu réactionnel est laissé une nuit à 40°C avant d'être

précipité dans 40ml d'éther diéthylique. Après filtration, on obtient 98mg d'une poudre blanche (Rendement : 84%) $C_{34}H_{48}N_{10}O_{11}$ ; m/z = 773 ($ES^{+}$)

Etape 2

95mg de l'intermédiaire obtenu à l'étape 1 (0,12mmol ; 1équiv) et 430mg (0,15mmol, 1,25 eq) de 1,2-distearoyl-*sn*-glycero-3-phosphoethanolamine-N-[amino(polyethylene glycol)-2000] (ammonium salt) sont dissous dans 3 mL de DMSO séché sur tamis en présence de 25µl de triéthylamine. Le milieu réactionnel est agité 48h à température ambiante avant d'être précipité dans 40ml d'éther diéthylique. Après filtration, on obtient 400mg d'une poudre blanche. Le produit ainsi obtenu est ensuite purifié par flash sur cartouche C4 avec un gradient formiate d'ammonium 10mM pH6 / Methanol. On obtient 260mg d'une poudre blanche (Rendement : 62%). $C_{164}H_{305}N_{12}O_{64}P$ ; MALDI-TOF: Mode positif m/z = 3501

Autre exemple :

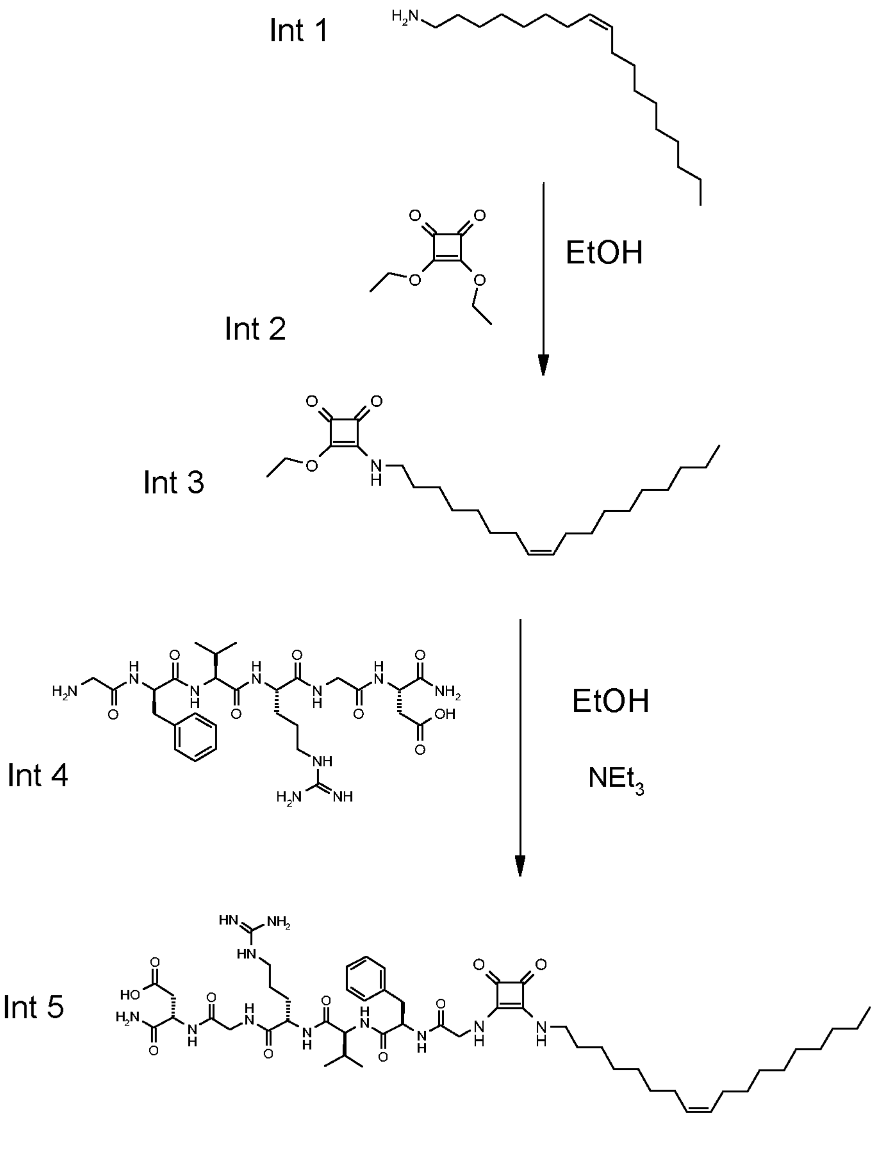


**Exemple 5 :** Synthèse d'un peptide lipophile RGD cyclique

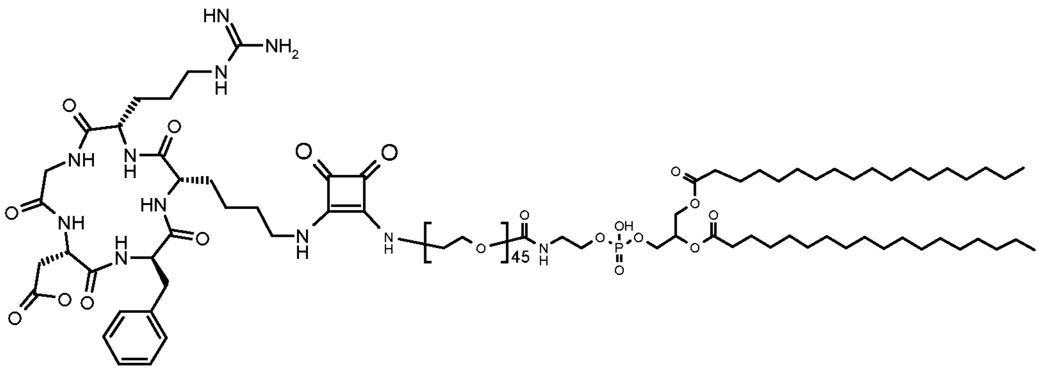

Même mode opératoire que pour l'exemple 4 en partant de 90mg de peptide cyclique RGDfK acheté chez Bachem. $C_{163}H_{302}N_{11}O_{63}P$ ; MALDI-TOF: Mode positif m/z = 3456

**Exemple 6 :** Synthèse d'un lipophile avec un peptidomimétique du RGD ; exemple de composé naphtyridine

Schéma de synthèse :

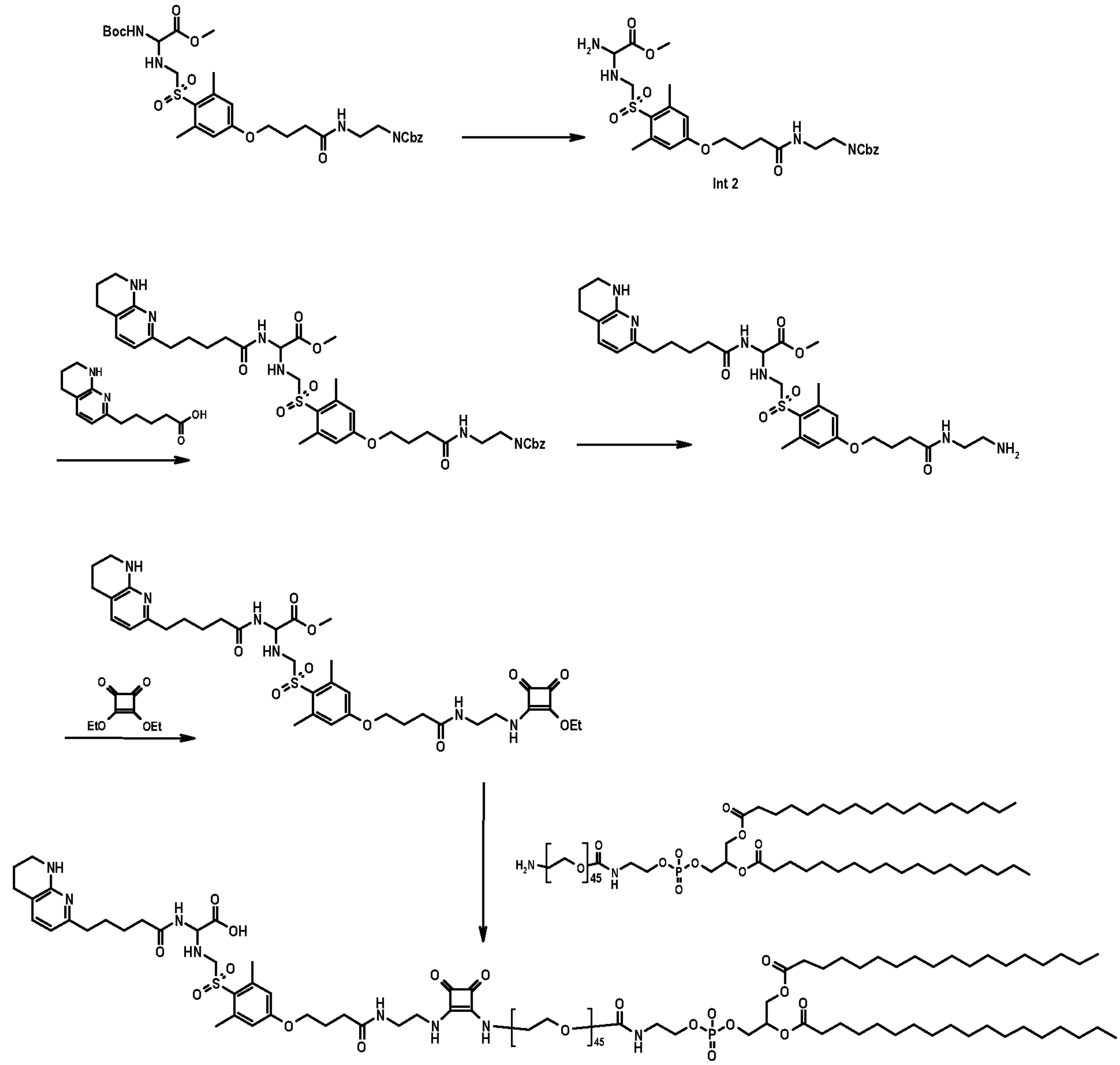


Etape 1 :

1g de l'int 1 est dissout dans 5 ml de CH2Cl2. 5ml de TFA sont ajoutés au milieu. Laisser 3h à TA puis évaporer à sec. Reprendre dans 2*40 ml d'éther iso, récupérer une huile que l'on sèche par évaporation.
$m_{obt}$ = 0,8 g ; Rdt = 90% ; $C_{26}H_{36}N_{4O8}S$ ; MALDI-TOF: Mode positif m/z = 564

Etape 2 :

| Réactifs | Quantités | Solvants |
|---|---|---|
| Int 2 | M = 0.564g (0.001 ml) | |
| Int 3 | M = 0.235g (0.00023 ml) | |
| HOBT | M = 0.131g | |

(suite)

| Réactifs | Quantités | Solvants |
|---|---|---|
| DIPEA | M = 0.286g | DMF V = 10 ml |
| EDCI | V = 0.2 ml | |
| Int.4 | | |

Dissoudre l'acide dans le DMF puis introduire HOBT et EDCI et laisser 1h sous argon. Rajouter l'int 2 et la DIPEA ; laisser 18h à TA sous argon. Après évaporation l'huile est reprise dans $CH_2Cl_2$ et lavée par une solution diluée de $Na_2CO_3$ ; après évaporation, obtention d'une huile.
$m_{obt}$ = 0,600 g; Rdt =77%; $C_{39}H_{52}N_6O_9S$ ; M/Z = 780

Etape 3

| Réactifs | Quantités | Solvants |
|---|---|---|
| Int 4 | M = 0.6g (0.0077ml) | MeOH V = 30 ml |
| Pd/C 10 % | 1 pointe de spatule | |
| Int. 5 | | |

Dissoudre l'int 4 dans le méthanol et introduire la solution dans l'autoclave de 125 ml ; rajouter le catalyseur et laisser 3h sous pression d'hydrogène (P= 5 bars) à 30°C.

Après filtration du catalyseur et évaporation, obtention d'une huile que l'on lave par 50 ml d'éther iso.
$m_{obt}$ = 0,300 g ; Rdt =60% ; $C_{31}H_{46}N_6O_7S$ ; HPLC = 90% ; M/Z = 646

Etape 4:

| Réactifs | Quantités | Solvants |
|---|---|---|
| Int 5 | M = 0.300g (0.000442 ml) | DMSO V = 10 ml |
| Diethyl squarate | M = 0.286g | |
| Int.6 | | TEA V = 0.25 ml |

Dissoudre l'int 5 dans le DMSO puis rajouter le diethyl squarate et quelques gouttes de TEA ; Laisser 1 nuit à TA sous argon. Verser dans de l'éther : obtention d'une pâte blanche.
$m_{obt}$ = 0,330 g ; Rdt =97%; $C_{37}H_{50}N_6O_{10}S$ ; M/Z = 770

Etape 5:

| Réactifs | Quantités | Solvants |
|---|---|---|
| Int. 6 | M = 0.330g (0.00043 ml) | DMSO V = 10 ml |
| DSPE-$PEG_{2000}$-$NH_2$ | M = 1.07g (0.000385 ml) | |
| Solution saturée de $Na_2CO_3$ | M = 0.131g | |
| Int.7 | | |

Dissoudre l'int 6 et la DSPE-PEG2000-NH2 dans le DMSO, rajouter 3 gouttes de solution saturée en Na2CO3 et 2ml d'H2O. Le milieu réactionnel est agité à température ambiante pendant 48h et est précipité dans l'éther. La pâte obtenue est solubilisée dans du méthanol puis elle est purifiée sur silice, éluant CH2Cl2. Après avoir rassemblé et évaporé les fractions conformes : obtention de cristaux.

REMARQUE : Le produit obtenu est sous la forme acide par coupure de l'ester de méthyle par la présence de Na2CO3.
$m_{obt}$ = 0,170 g ;Rdt =17% ; $C_{166}H_{308}N_9O_{63}PS$ ; M/Z = 3500

**Exemple 7 :**

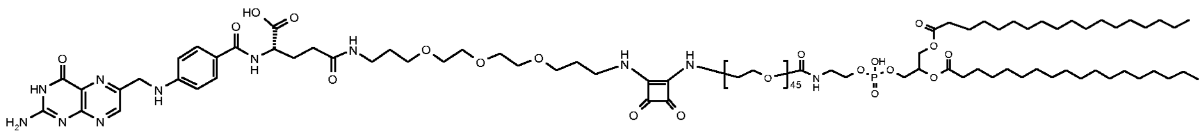

Etape 1 :

150mg du composé e) de l'exemple 11 de la demande de brevet WO 2004/112839 sont mis en réaction avec 35μl de diethylsquarate selon le même mode opératoire qu'à l'étape 4 de l'exemple 6 de ce brevet.
$C_{35}H_{45}N_9O_{11}$ ; m/z ($ES^-$) = 766

Etape 2 :

Le composé obtenu à l'étape 1 est mis en réaction avec 440mg de DSPE-PEG2000-NH2 comme décrit à l'étape 5 de l'exemple 6.
$C_{165}H_{302}N_{11}O_{64}P$ ; m/z ($ES^-$) = 3493

**Exemple 8 :**

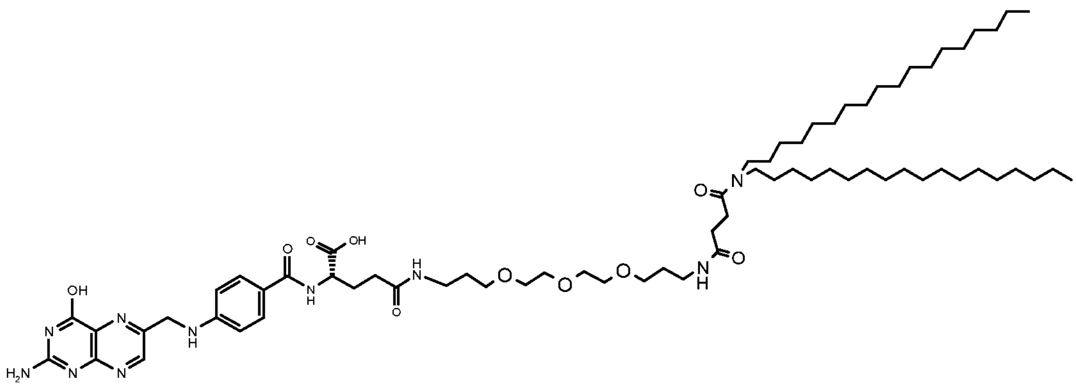

0.517g du composé e) de l'exemple 11 de la demande de brevet WO 2004/112839 sont mis en réaction avec 500mg du composé issu de l'étape 2 de l'exemple 3.2 dans 5ml de DMSO, 93mg de NHS et 166mg de DCC. Après 24h à température ambiante, le milieu réactionnel est précipité dans 50ml d'eau et filtré. Après séchage sous vide, 515mg d'une poudre jaune sont obtenus.
$C_{69}H_{118}N_{10}O_{10}$ ; m/z ($ES^-$) = 1246

**Exemple 9 :**

Etape 1 :

200mg de peptide cyclique 8-amino-3,6-dioxaoctanoylcyclo-Cys-Met-Lys(TFA)-Thr-Asp-Thr-Arg-Leu-Cys-COOH synthétisés par Polypeptide sont mis en réaction dans 1ml de DMSO et 0.23µl de diethylsquarate selon le même mode opératoire qu'à l'étape 4 de l'exemple 6 de ce brevet.
$C_{55}H_{87}F_3N_{14}O_{21}S_3$ ; m/z ($ES^-$) = 1432

Etape 2 :

Le composé obtenu à l'étape 1 est mis en réaction avec 362mg de DSPE-PEG2000-NH2 comme décrit à l'étape 5 de l'exemple 6.
$C_{185}H_{344}F_3N_{16}O_{74}PS_3$ ; m/z ($ES^-$) = 4160

Etape 3 :

Le composé obtenu à l'étape précédente est mis en solution dans la pipéridine 0.2M dans du méthanol pendant 3 heures à 0°C.
$C_{183}H_{345}N_{16}O_{73}PS_3$ ; m/z ($ES^-$) = 4064

**Exemple 10 :**

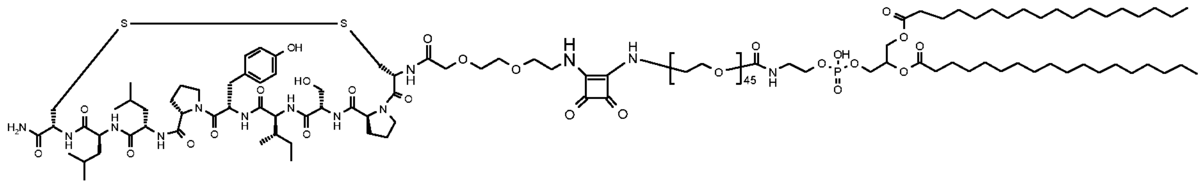

Etape 1 :

200mg de peptide cyclique 8-amino-3,6-dioxaoctanoylcyclo-Cys-Pro-Ser-Ile-Tyr-Pro-Leu-Leu-Cys-NH2 synthétisés par Polypeptide sont mis en réaction dans 1ml de DMSO et 0.23µl de diethylsquarate selon le même mode opératoire qu'à l'étape 4 de l'exemple 6 de ce brevet.
$C_{58}H_{87}N_{11}O_{17}S_2$; m/z ($ES^-$) = 1273

Etape 2 :

Le composé obtenu à l'étape 1 est mis en réaction avec 350mg de DSPE-PEG2000-NH2 comme décrit à l'étape 5 de l'exemple 6.
$C_{188}H_{344}N_{13}O_{70}PS_2$ ; m/z ($ES^-$) = 4000

**Exemple 11** :

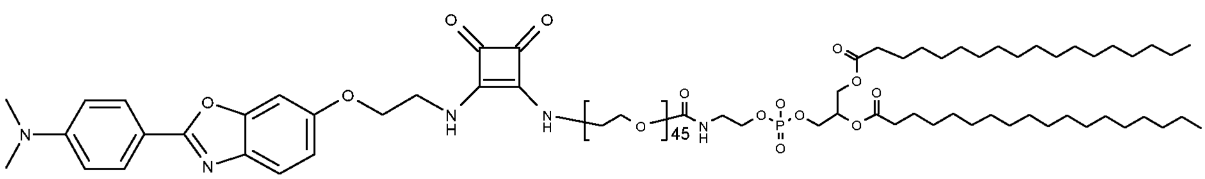

Etape 1 :

100mg de {4-[6-(2-amino-ethoxy)-benzooxazol-2-yl]-phényle}-diméthyle-amine sont mis en réaction avec 50μl de diethylsquarate selon le même mode opératoire qu'à l'étape 4 de l'exemple 6 de ce brevet.
$m_{obt}$ = 43 mg ; Rdt =34% ; $C_{23}H_{23}N_3O_5$ ; m/z ($ES^-$) = 421

Etape 2 :

Le composé obtenu à l'étape 1 est mis en réaction avec 256mg de DSPE-PEG2000-$NH_2$ comme décrit à l'étape 5 de l'exemple 6.
$m_{obt}$ = 182 mg ; Rdt =63% ; $C_{153}H_{280}N_5O_{58}P$ ; m/z ($ES^-$) = 3148

**Exemple 12 :** Synthèse d'une émulsion contenant le composé de l'exemple 1 et du PEG-2000

10 g de Miglyol®, 420 mg de Lipoïd S75 (Lipoïd GMBH), 30 mg de DSPE-PEG-2000 (Lipoïd) et 150 mg du composé de l'exemple 1 sont dissous dans un mélange chloroforme/méthanol (90/10). Ceci représente un taux de 6% massique de tensioactifs totaux par rapport à la masse d'huile utilisée.

Ce mélange est passé à l'évaporateur rotatif afin d'éliminer les solvants. On obtient une phase huileuse parfaitement homogène. 40 ml d'eau à 2,5 % m/m de glycérol sont ajoutés puis pré-émulsionnés à l'aide d'un Ultraturax. La pré-émulsion est ensuite finalisée au microfluidiseur (Microfluidics M-110-S) en recyclant pendant 3 à 4 minutes, ce qui correspond à environ 25 passages dans la cellule.

Le taux massique de tensioactifs totaux par rapport à la solution finale est de 1.18% (0.6/50.6) Le pH est contrôlé puis ajusté à ~7. L'émulsion est filtrée sur 0,45 μ. De la Gentalline est ajoutée à raison de 7 μl/100 ml afin de garantir la conservation.

Le diamètre hydrodynamique (Zetasizer de chez Malvern) de l'émulsion obtenue est de 190 nm.

**Exemple 13 :** Synthèse d'une émulsion contenant le composé de l'exemple 2

3 g de Miglyol®, et 180 mg du composé de l'exemple 2 sont dissous dans un mélange Chloroforme / méthanol (90/10). Ceci représente un taux de 6% massique de tensioactifs totaux par rapport à la masse d'huile utilisée. Ce mélange est passé à l'évaporateur rotatif afin d'éliminer les solvants. On obtient une phase huileuse parfaitement homogène. 27 ml d'eau à 2,5 % m/m de glycérol sont ajoutés puis pré-émulsionnés à l'aide d'un Ultraturax. La pré-émulsion est ensuite finalisée au microfluidiseur (Microfluidics M-110-S) en recyclant pendant 3 à 4 minutes, ce qui correspond à environ 25 passages dans la cellule.

Le taux massique de tensioactifs totaux par rapport à la solution finale est de 0.6% (0.18/30.18) Le pH est contrôlé puis ajusté à ~7. L'émulsion est filtrée sur 0,45 μ. De la Gentalline est ajoutée à raison de 7 μl/100 ml afin de garantir la conservation.

Le diamètre hydrodynamique (Zetasizer de chez Malvern) de l'émulsion obtenue est de 160 nm.

**Exemple 14** : synthèse d'une émulsion contenant le composé de l'exemple 3 et du PEG-5000 (émulsion à 20% d'huile)

10 g de Miglyol®, 420 mg de Lipoïd S75 (Lipoïd GMBH), 30 mg de DSPE-PEG-5000 (Lipoïd) et 150 mg du composé de l'exemple 3 sont dissous ans un mélange chloroforme/méthanol (90/10). Ceci représente un taux de 6% massique de tensioactifs totaux par rapport à la masse d'huile utilisée.

Ce mélange est passé à l'évaporateur rotatif afin d'éliminer les solvants. On obtient une phase huileuse parfaitement homogène.

40 ml d'eau à 2,5 % m/m de glycérol sont ajoutés puis pré-émulsionnés à l'aide d'un Ultraturax. La pré-émulsion est ensuite finalisée au microfluidiseur (Microfluidics M-110-S) en recyclant pendant 3 à 4 mn , ce qui correspond à environ 25 passages dans la cellule.

Le taux massique de tensioactifs totaux par rapport à la solution finale est de 1.2%.(0.6/50.6) Le pH est contrôlé puis ajusté à ~7. L'émulsion est filtrée sur 0.45 μ. De la Gentalline est ajoutée à raison de 7 μl/100 ml afin de garantir la conservation. Le diamètre hydrodynamique (Zetasizer de chez Malvern) de l'émulsion obtenue est de 210 nm.

**Exemple 15** : Synthèse d'une émulsion vectorisée RGD contenant du DSPE-PEG-2000 et du composé de l'exemple 1 (émulsion à 20% d'huile)

10 g de Miglyol®, 400 mg d'Egg phosphatidylcholine (EPC, Lipoïd GMBH), 110 mg de DSPE-PEG-2000 (Lipoïd), 210 mg du composé de l'exemple 1 et 60 mg du composé de l'exemple 4 sont dissous dans un mélange chloroforme/méthanol (90/10). Ceci représente un taux de 7.8 % massique de tensioactifs totaux par rapport à la masse d'huile utilisée.

Ce mélange est passé à l'évaporateur rotatif afin d'éliminer les solvants. On obtient une phase huileuse (phase lipidique constituée d'huile) parfaitement homogène.

40 ml d'eau à 2,5 % m/m de glycérol sont ajoutés puis pré-émulsionnés à l'aide d'un Ultraturax. La pré-émulsion est ensuite finalisée au microfluidiseur (Microfluidics M-110-S) en recyclant pendant 3 à 4 mn , ce qui correspond à environ 25 passages dans la cellule.

Le taux massique de tensioactifs totaux par rapport à la solution finale est de 1.54%.

Le pH est contrôlé puis ajusté à ~7. L'émulsion est filtrée sur 0,45 μ. De la Gentalline est ajoutée à raison de 7 μl/100 ml afin de garantir la conservation.

Le diamètre hydrodynamique (Zetasizer de chez Malvern) de l'émulsion obtenue est de 168 nm. Un suivi du diamètre de l'émulsion est réalisée par diffusion dynamique de la lumière (Zetasizer de chez Malvern) pendant un an avec une conservation de l'émulsion à 4°C.

Le diamètre hydrodynamique à 1 an est de 170 nm.

**Exemple 16** : Synthèse d'une émulsion vectorisée RGD contenant du composé de l'exemple 1, du DSPE-PEG-2000 et de la rhodamine (émulsion à 20% d'huile)

10 g de Miglyol®, 330 mg de Lipoïd S75 (Lipoïd GMBH), 30 mg de DSPE-PEG-2000 (Lipoïd) et 150 mg du composé de l'exemple 1, 90 mg du composé de l'exemple 4 et 2 mg de DSPE-rhodamine sont dissous dans un mélange chloroforme / méthanol (90/10). Ceci représente un taux de 6% massique de tensioactifs totaux par rapport à la masse d'huile utilisée.

Ce mélange est passé à l'évaporateur rotatif afin d'éliminer les solvants. On obtient une phase huileuse parfaitement homogène.

40 ml d'eau à 2,5 % m/m de glycérol sont ajoutés puis pré-émulsionnés à l'aide d'un Ultraturax. La pré-émulsion est ensuite finalisée au microfluidiseur (Microfluidics M-110-S) en recyclant pendant 3 à 4 mn , ce qui correspond à environ 25 passages dans la cellule.

Le taux massique de tensioactifs totaux par rapport à la solution finale est de 1.2%

Le pH est contrôlé puis ajusté à ~7. L'émulsion est filtrée sur 0.45 μ. De la Gentalline est ajoutée à raison de 7 μl/100 ml afin de garantir la conservation.

Le diamètre hydrodynamique (Zetasizer de chez Malvern) de l'émulsion obtenue est de 206 nm.

**Exemple 17** : Synthèse d'une émulsion vectorisée RGD contenant du composé de l'exemple 1, du DSPE-PEG-2000 et de la rhodamine.

10g de Miglyol®, 330 mg d'Egg phosphatidylcholine (EPC, Lipoïd GMBH), 30 mg de DSPE-PEG-2000 (Lipoïd), 150 mg du composé de l'exemple 1, 90 mg du composé de l'exemple 5 et 2 mg de DSPE-rhodamine sont dissous dans un mélange chloroforme / méthanol (90/10). Ceci représente un taux de 6 % massique de tensioactifs totaux par rapport à la masse d'huile utilisée.

Ce mélange est passé à l'évaporateur rotatif afin d'éliminer les solvants. On obtient une phase huileuse parfaitement homogène.

40 ml d'eau à 2,5 % m/m de glycérol sont ajoutés puis pré-émulsionnés à l'aide d'un Ultraturax. La pré-émulsion est ensuite finalisée au microfluidiseur (Microfluidics M-110-S) en recyclant pendant 3 à 4 mn , ce qui correspond à environ 25 passages dans la cellule.

Le taux massique de tensioactifs totaux par rapport à la solution finale est de 1.2%.

Le pH est contrôlé puis ajusté à ~7. L'émulsion est filtrée sur 0,45 μ. De la Gentalline est ajoutée à raison de 7 μl/100 ml afin de garantir la conservation.

Le diamètre hydrodynamique (Zetasizer de chez Malvern) de l'émulsion obtenue est de 206 nm.

**Exemple 18 :** Mesures de relaxivité

Les mesures de relaxivité sont réalisées sur des Relaxomètres Minispec à 20 et 60 MHz.

La solution mère est diluée sur 6 points de gamme dans l'eau milli Q afin de pouvoir étudier la linéarité des vitesses de relaxation en fonction de la concentration. La gamme de concentrations va de 0.1 à 2.5 mM en Gd.

La mesure de relaxivité est effectuée à 37°C. Le dosage de Gd est réalisé par ICP-AES sur tous les points de la gamme.

| Emulsion | 20 MHz | | 60MHz | |
|---|---|---|---|---|
| | r1($mM^{-1}s^{-1}$) | r2($mM^{-1}s^{-1}$) | r1($mM^{-1}s^{-1}$) | r2($mM^{-1}s^{-1}$) |
| **Exemple 7** | 16 | 19 | 14 | 20 |
| **Exemple 8** | 39 | 43 | 29 | 52 |
| **Exemple 9** | 18 | 22 | 16 | 20 |
| **Exemple 10** | 23 | 25 | 24 | 35 |
| **Exemple 12** | 27 | 28 | 24 | 34 |

**Exemple 19 :** tests de toxicité de l'émulsion de l'exemple 10

**Test *in vivo* :**

Sur Souris « swiss » d'environ 25g : Injection IV caudale vigile manuelle à 2mL/min en isovolume (200µl/animal soit 6,67ml/kg).

A 24h : anesthésie à l'isoflurane, prélèvement sublingual pour bilan hématologie sur l'automate MS4 puis exsanguination avec seringue + aiguilles héparinées.

Symptomatologie: Pas de létalité de constatée ni de signes cliniques délétères à la dose et délai testés.

Hématologie : bilan hématologique normal.

Evolution du poids des souris après injection : pas de variation pondérale significative.

**Test *in vitro*** : test MTT sur L929 à 24h.

La CEL50 est supérieure à la dose testée qui est de 3 mM en Gd.

**Exemple 20 :** Mesure de l'IC50

La mesure d'IC50 des émulsions est réalisée sur des cellules HUVEC surexprimant ∞vβ3 par des mesures de compétitions avec de l'Echistatine $^{125}I$.

La suspension des HUVEC est distribuée dans une plaque 96 puits à fond conique, à raison de $2.10^5$ cellules dans 50 µL en tampon de binding. Cinquante µL des solutions de concentration croissante d'échistatine ou des produits RGD sont ajoutés par puits. Le contrôle positif est réalisé par l'ajout de tampon de binding sans compétiteur. L'ensemble des points de concentration est réalisé en duplicat. La plaque est incubée 2 h à température ambiante sous agitation. Cinquante µL de la solution d'échistatine-$^{125}I$-SIB à 3 nM sont alors distribués dans chaque puits et la plaque est de nouveau incubée 2 h à température ambiante sous agitation. Les mélanges réactionnels sont transférés dans des ampoules contenant 200 µL d'un coussin de densité composé de paraffine et de dibutylphtalate (10/90). Les microtubes sont ensuite centrifugés à 12 000 rpm pendant 3 min. Les tubes sont finalement congelés dans l'azote liquide, puis sectionnés afin de compter le culot cellulaire et le surnageant au compteur gamma. Une courbe de compétition est alors tracée où la fixation relative de l'échistatine$^{125}I$-SIB est déterminée par l'équation suivante :

$$\text{Fixation relative de l'échistatine-}I^{125}\text{-SIB} = \frac{\text{Radioactivité fixée en présence de compétiteur (cpm)}}{\text{Radioactivité de l'échantillon contrôle (cpm)}} \times 100$$

Les données sont analysées à l'aide du logiciel GraphPad Prism® 5.0 qui détermine les valeurs $IC_{50}$ pour chaque produit à partir de la courbe de compétition.

| Composé | IC50 (nM de ligand de cliblage | IC50 (nM d'émulsion |
|---|---|---|
| **Exemple 4** | 300 | |
| **Exemple 5** | 1 | |
| **Exemple 6** | 0.4 | |
| **Exemple 10** | 4 500 | 2 |

(suite)

| Composé | IC50 (nM de ligand de ciblage | IC50 (nM d'émulsion |
|---|---|---|
| **Exemple 11** | 13 500 | 2.1 |

## Revendications

**1.** Composition de nanoémulsion huile dans eau pour IRM, comprenant :

- une phase aqueuse, représentant 70 à 90% en poids de la composition, avantageusement 75 à 85%, plus avantageusement de 78 à 82%
- une phase lipidique comprenant une huile, représentant 9.5 à 29.5% en poids de la composition, avantageusement 14 à 25%, plus avantageusement 17 à 21%,
- un tensioactif à l'interface entre les phases aqueuse et lipidique, le tensioactif comprenant au moins un chélate de métal paramagnétique amphiphile, au moins un ligand de ciblage amphiphile et éventuellement un lipide amphiphile ;
- la teneur totale en tensioactif en poids par rapport à l'huile étant comprise entre 4 et 10%, avantageusement entre 5 et 8 % ;
- la teneur totale en tensioactif en poids par rapport à la composition étant comprise entre 0.35 et 2.95%, avantageusement entre 0.5 et 2 % ;
- l'huile comprenant au moins 70%, avantageusement au moins 80%, de façon avantageuse au moins 95% en poids, notamment au moins 97 %, d'acides gras saturés en C6-C18, avantageusement en C6-C14, plus avantageusement en C6-C10.

**2.** Composition selon la revendication 1, **caractérisée en ce que** le ligand de ciblage amphiphile compris dans le tensioactif représente de 0,01 à 10% en poids du total de tensioactif, avantageusement 0.05 à 5 %, notamment 0.05 à 1 %.

**3.** Composition selon la revendication 1 ou 2, **caractérisée en ce que** le tensioactif représente 5 à 8% en poids de l'huile.

**4.** Composition selon la revendication 1 à 3, **caractérisée en ce que** les acides gras saturés se trouvent sous la forme de triglycérides d'acides gras saturés.

**5.** Composition selon la revendication 1 à 3, **caractérisée en ce que** l'huile comprend des acides gras saturés dans les proportions suivantes:

- en C6-C18 > 70%, avantageusement > 80%, plus avantageusement >95%, encore plus avantageusement > 98%
- ou en C6-C14 > 70%, avantageusement > 80%, plus avantageusement >95%, encore plus avantageusement > 98%
- ou en C8+C10 > 70%, avantageusement > 80%, plus avantageusement >95%, encore plus avantageusement > 98%.

**6.** Composition selon l'une quelconque des revendications 1 à 5, **caractérisée en ce que** le tensioactif comprend :

- 50 à 95% en poids de lipide amphiphile,
- 5 à 50% en poids de chélate de métal paramagnétique amphiphile, avantageusement 5 à 30% en poids, et le cas échéant
- 0.05 à 5% en poids d'un ligand de ciblage amphiphile.

**7.** Composition selon l'une quelconque des revendications 1 à 5, **caractérisée en ce que** le tensioactif comprend 95 à 99.95% de chélate de métal paramagnétique amphiphile et 0.05 à 5% d'un ligand de ciblage amphiphile.

**8.** Composition selon l'une quelconque des revendications 1 à 7, **caractérisée en ce que** le chélate du de métal paramagnétique amphiphile est un chélate macrocyclique choisi parmi : DOTA, DO3A, HPDO3, BTDO3A, PCTA, DOTAM, DOTMA, DOTA-GA, AAZTA, HOPO, ainsi que leurs multimères et leurs dérivés.

**9.** Composition selon l'une quelconque des revendications 1 à 8, **caractérisée en ce que** le ligand de ciblage est un biovecteur choisi parmi : les peptides (avantageusement de moins de 20 acides aminés, plus avantageusement de 5 à 10 acides aminés), les pseudopeptides, les peptidomimétiques, les acides aminés, les agents de ciblage d'intégrines, les glycoprotéines, les lectines, la biotine, les dérivés ptéroïques ou aminoptéroïques, les dérivés de l'acide folique et antifolique, les anticorps ou fragments d'anticorps, l'avidine, les stéroïdes, les oligonucléotides, les séquences d'acide ribonucléique, les séquences d'acide désoxyribonucléique, les hormones, les protéines éventuellement recombinantes ou mutées, les mono ou polysaccharides, les composés à squelette benzothiazole, benzofurane, styrylbenzoxazole/thiazole/imidazole / quinoline, styrylpiridine.

**10.** Composition selon l'une quelconque des revendications 1 à 9 **caractérisée en ce que** le lipide amphiphile est un phospholipide, avantageusement choisi parmi la phosphatidylcholine la dioléoylphosphatidylcholine, la dimyristoylphosphatidylcholine, la dipalmitoylphosphatidylcholine, la distéaroylphosphatidylcholine, la phosphatidyléthanolamine, la sphingomyéline, la phosphatidylsérine, le phosphatidylinositol, la lécithine.

**11.** Composition selon l'une quelconque des revendications 1 à 10 **caractérisée en ce que** le ligand de ciblage amphiphile est de formule Bio - L - Lipo, dans laquelle :

- Bio est la partie de reconnaissance biologique localisée à la surface externe des nanogouttelettes
- Lipo est un groupe lipophile permettant l'insertion du biovecteur lipophile au sein de la couche de tensioactif
- L est un groupe de liaison reliant Bio et Lipo, avantageusement choisi parmi :

  - rien ou une simple liaison, squarate, C1-6 alkylène, PEG par exemple CH2-(CH2-O-CH2)k-CH2 avec k=1 à 10, $(CH_2)_3$-NH, NH-$(CH_2)_2$-NH, NH-$(CH_2)_3$-NH, , $(CH_2)_n$, $(CH_2)_n$-CO-, -$(CH_2)_n$NH-CO- avec n = 2 à 10 , $(CH_2CH_2O)_q(CH_2)_r$-CO- , $(CH_2CH_2O)q(CH_2)_r$-NH-CO- avec q = 1-10 et r=2-10, $(CH_2)_n$-CONH - , $(CH_2)_n$-CONH - PEG, $(CH_2)_n$-NH- HOOC-$CH_2$-O-$(CH_2)_2$-O-$(CH_2)_2$-O-$CH_2$-COOH ; HOOC-$(CH_2)_2$-$CO_2$-$(CH_2)_2$-OCO-$(CH_2)_2$-COOH ; HOOC-CH(OH)-CH(OH)-COOH; HOOC-$(CH_2)_n$-COOH; $NH_2$-$(CH_2)_n$-$NH_2$, avec n=0-20; $NH_2$-$(CH_2)_n$-$CO_2H$ ; $NH_2$-$CH_2$ - ($CH_2$-O-$CH_2)_n$-$CO_2H$ avec n=1 à 10,
  - P1-1-P2, identiques ou différents, P1 et P2 étant choisis parmi O, S, NH, rien, $CO_2$, NHCO, CONH, NHCONH, NHCSNH, $SO_2$NH-, $NHSO_2$-, squarate

avec 1 = alkyle, alkoxyalkyle, polyalkoxyalkyle (PEG), alkyle interrompu par un ou plusieurs squarates ou par un ou plusieurs aryles, avantageusement phényles, alcényle, alcynyle, alkyle interrompus par un ou plusieurs groupes choisis parmi -NH-, -O-, -CO-, -NH(CO)-, -(CO)NH-, -O(CO)-, ou-(OC)O- .

**12.** Composition selon l'une quelconque des revendications 1 à 11, **caractérisée en ce que** le tensioactif comprend en outre un agent de furtivité amphiphile, avantageusement un dérivé PEG, un dérivé ganglioside, un polysaccharide.

**13.** Composition selon l'une quelconque des revendications 1 à 12, **caractérisée en ce que** le métal paramagnétique du chélate de métal paramagnétique amphiphile est choisi parmi : manganèse, fer, cobalt, nickel, cuivre, molybdène, ruthénium, cérium, praseodymium, neodymium, prométhium, samarium, europium, gadolinium, terbium, dysprosium, holmium, erbium, thulium, et ytterbium ; avantageusement parmi Gd(III), Mn(II), europium et dysprosium.

**14.** Procédé de préparation d'une composition selon l'une quelconque des revendications 1 à 13, comprenant les étapes :

- préparation d'une phase lipidique comprenant

  ◦ éventuellement un premier tensioactif lipide amphiphile
  ◦ une huile comprenant au moins 70% en poids, avantageusement au moins 80%, plus avantageusement au moins 95 % d'acides gras saturés en C6-C18, avantageusement en C6-C14, plus avantageusement en C6-C10
  ◦ un chélate de métal paramagnétique amphiphile
  ◦ un ligand de ciblage amphiphile

- dispersion de la phase lipidique dans une solution aqueuse de manière à former une nanoémulsion huile dans eau
- récupération de la composition de nanoémulsion obtenue.

**15.** Composition selon la revendication 1 à 13, pour son utilisation dans le diagnostic, avantageusement de maladies cancéreuses, inflammatoires, neurodégénératives et/ou cardiovasculaires.

## Patentansprüche

**1.** Zusammensetzung einer Öl-in-Wasser-Nanoemulsion für MRI, umfassend:

- eine wässrige Phase, die 70 bis 90 Gew.-% der Zusammensetzung, vorteilhaft 75 bis 85 Gew.-%, vorteilhafter 78 bis 82 Gew.-% darstellt,
- eine Lipidphase, umfassend ein Öl, die 9,5 bis 29,5 Gew.-% der Zusammensetzung, vorteilhaft 14 bis 25 Gew.-%, vorteilhafter 17 bis 21 Gew.-% darstellt,
- ein Tensid an der Grenzfläche zwischen der wässrigen Phase und der Lipidphase, wobei das Tensid mindestens ein amphiphiles paramagnetisches Metallchelat, mindestens einen amphiphilen Targeting-Liganden und gegebenenfalls ein amphiphiles Lipid umfasst;
- wobei der Gesamtgewichtsgehalt am Tensid in Bezug auf das Öl zwischen 4 und 10 %, vorteilhaft zwischen 5 und 8 % beträgt;
- wobei der Gesamtgewichtsgehalt am Tensid in Bezug auf die Zusammensetzung zwischen 0,35 und 2,95 %, vorteilhaft zwischen 0,5 und 2 % beträgt;
- wobei das Öl mindestens 70 Gew.-%, vorteilhaft mindestens 80 Gew.-%, auf vorteilhafte Weise mindestens 95 Gew.-%, insbesondere mindestens 97 Gew.-% gesättigte C6-C18-, vorteilhaft C6-C14-, vorteilhafter C6-C10-Fettsäuren umfasst.

**2.** Zusammensetzung nach Anspruch 1,
**dadurch gekennzeichnet, dass** der amphiphile Targeting-Ligand, der in dem Tensid enthalten ist, 0,01 bis 10 Gew.-% der Gesamtheit des Tensids, vorteilhaft 0,05 bis 5 Gew.-%, insbesondere 0,05 bis 1 Gew.-% darstellt.

**3.** Zusammensetzung nach Anspruch 1 oder 2,
**dadurch gekennzeichnet, dass** das Tensid 5 bis 8 Gew.-% des Öls darstellt.

**4.** Zusammensetzung nach Anspruch 1 bis 3,
**dadurch gekennzeichnet, dass** die gesättigten Fettsäuren in der Form von Triglyceriden gesättigter Fettsäuren vorliegen.

**5.** Zusammensetzung nach Anspruch 1 bis 3,
**dadurch gekennzeichnet, dass** das Öl gesättigte Fettsäuren in den folgenden Anteilen umfasst:

- C6-C18 > 70 %, vorteilhaft > 80 %, vorteilhafter > 95 %, noch vorteilhafter > 98 %
- oder C6-C14 > 70 %, vorteilhaft > 80 %, vorteilhafter > 95 %, noch vorteilhafter > 98 %
- oder C8+C10 > 70 %, vorteilhaft > 80 %, vorteilhafter > 95 %, noch vorteilhafter > 98 %.

**6.** Zusammensetzung nach einem der Ansprüche 1 bis 5,
**dadurch gekennzeichnet, dass** das Tensid umfasst:

- 50 bis 95 Gew.-% amphiphiles Lipid,
- 5 bis 50 Gew.-% amphiphiles paramagnetisches Metallchelat, vorteilhaft 5 bis 30 Gew.-%, und gegebenenfalls
- 0,05 bis 5 Gew.-% eines amphiphilen Targeting-Liganden.

**7.** Zusammensetzung nach einem der Ansprüche 1 bis 5,
**dadurch gekennzeichnet, dass** das Tensid 95 bis 99,95 % amphiphiles paramagnetisches Metallchelat und 0,05 bis 5 % eines amphiphilen Targeting-Liganden umfasst.

**8.** Zusammensetzung nach einem der Ansprüche 1 bis 7,
**dadurch gekennzeichnet, dass** das amphiphile paramagnetische Metallchelat ein makrozyklisches Chelat ist, ausgewählt aus: DOTA, DO3A, HPDO3, BTDO3A, PCTA, DOTAM, DOTMA, DOTA-GA, AAZTA, HOPO, sowie ihren Multimeren und ihren Derivaten.

**9.** Zusammensetzung nach einem der Ansprüche 1 bis 8,

**dadurch gekennzeichnet, dass** der Targeting-Ligand ein Biovektor ist, ausgewählt aus: Peptiden (vorteilhaft weniger als 20 Aminosäuren, vorteilhafter 5 bis 10 Aminosäuren), Pseudopeptiden, Peptidomimetika, Aminosäuren, Targeting-Agentien von Integrinen, Glycoproteinen, Lectinen, Biotin, Pterin- oder Aminopterin-Derivaten, Folsäure- und Antifolsäure-Derivaten, Antikörpern oder Antikörperfragmenten, Avidin, Steroiden, Oligonukleotiden, Ribonukleinsäuresequenzen, Desoxyribonukleinsäuresequenzen, Hormonen, eventuell rekombinanten oder mutierten Proteinen, Mono- oder Polysacchariden, Verbindungen mit einem Benzothiazol-, Benzofuran-, Styrylbenzoxazol/thiazol/imidazol/chinolin-, Styrylpyridin Gerüst.

**10.** Zusammensetzung nach einem der Ansprüche 1 bis 9,
**dadurch gekennzeichnet, dass** das amphiphile Lipid ein Phospholipid ist, vorteilhaft ausgewählt aus Phosphatidylcholin, Dioleoylphosphatidylcholin, Dimyristoylphosphatidylcholin, Dipalmitoylphosphatidylcholin, Distearoylphosphatidylcholin, Phosphatidylethanolamin, Sphingomyelin, Phosphatidylserin, Phosphatidylinosit, Lecithin.

**11.** Zusammensetzung nach einem der Ansprüche 1 bis 10,
**dadurch gekennzeichnet, dass** der amphiphile Targeting-Ligand die Formel Bio-L-Lipo aufweist, wobei:

- Bio der biologisch erkennende Teil ist, der an der Außenfläche von Nanotröpfchen lokalisiert ist
- Lipo eine lipophile Gruppe ist, welche die Insertion des lipophilen Biovektors in die Schicht des Tensids ermöglicht
- L eine Bindungsgruppe ist, die Bio und Lipo verbindet, vorteilhaft ausgewählt aus:
- nichts oder einer Einfachbindung, Squarat, C1-6-Alkylen, PEG, beispielsweise CH2-(CH2-O-CH2)k-CH2, wobei k = 1 bis 10, $(CH_2)_3$-NH, NH-$(C_2)_2$-NH, NH-$(CH_2)_3$-NH, $(CH_2)_n$, $(CH_2)_n$-CO-, -$(CH_2)_n$NH-CO-, wobei n = 2 bis 10, $(CH_2CH_2O)_q(CH_2)_r$-CO-, $(CH_2CH_2O)$q$(CH_2)_r$-NH-CO-, wobei q = 1 bis 10 und r = 2 bis 10, $(CH_2)_n$CONH$^-$, $(CH_2)_n$CONH-PEG, $(CH_2)_n$-NH-HOOC-$CH_2$-O-$(CH_2)_2$-O$(CH_2)_2$-O-$CH_2$-COOH; HOOC-$(CH_2)_2$-$CO_2$-$(CH_2)_2$-OCO-$(CH_2)_2$-COOH; HOOC-CH(OH)-CH(OH)-COOH; HOOC-$(CH_2)_n$-COOH; $NH_2$-$(CH_2)_n$-$NH_2$, wobei n = 0 bis 20; $NH_2$-$(CH_2)_n$-$CO_2H$; $NH_2$-$CH_2$-$(CH_2$-O-$CH_2)_n$-$CO_2H$, wobei n = 1 bis 10,
- P1-1-P2, identisch oder verschieden, wobei P1 und P2 ausgewählt sind aus O, S, NH, nichts, $CO_2$, NHCO, CONH, NHCONH, NHCSNH, $SO_2$NH-, $NHSO_2$-, Squarat, wobei 1 = Alkyl, Alkoxyalkyl, Polyalkoxyalkyl (PEG), Alkyl, unterbrochen durch ein oder mehrere Squarate oder durch ein oder mehrere Aryle, vorteilhaft Phenyle, Alkenyl, Alkynyl, Alkyl, unterbrochen durch eine oder mehrere Gruppen, ausgewählt aus -NH-, -O-, -CO-, -NH(CO)-, -(CO)NH-, -O(CO)-oder -(OC)O-.

**12.** Zusammensetzung nach einem der Ansprüche 1 bis 11,
**dadurch gekennzeichnet, dass** das Tensid außerdem ein amphiphiles Verflüchtigungsmittel, vorteilhaft ein PEG-Derivat, ein Gangliosid-Derivat, ein Polysaccharid umfasst.

**13.** Zusammensetzung nach einem der Ansprüche 1 bis 12,
**dadurch gekennzeichnet, dass** das paramagnetische Metall des amphiphilen paramagnetischen Metallchelats ausgewählt ist aus: Mangan, Eisen, Kobalt, Nickel, Kupfer, Molybdän, Ruthenium, Cer, Praseodym, Neodym, Promethium, Samarium, Europium, Gadolinium, Terbium, Dysprosium, Holmium, Erbium, Thulium und Ytterbium; vorteilhaft aus Gd(III), Mn(II), Europium und Dysprosium.

**14.** Verfahren zur Herstellung einer Zusammensetzung nach einem der Ansprüche 1 bis 13, umfassend die Schritte:

- Herstellen einer Lipidphase, umfassend

  ◦ eventuell ein erstes amphiphiles Lipidtensid
  ◦ ein Öl, umfassend mindestens 70 Gew.-%, vorteilhaft mindestens 80 Gew.-%, vorteilhafter mindestens 95 Gew.-% gesättigte C6-C18-, vorteilhaft C6-C14-, vorteilhafter C6-C10-Fettsäuren
  ◦ ein amphiphiles paramagnetisches Metallchelat
  ◦ einen amphiphilen Targeting-Liganden

- Dispergieren der Lipidphase in einer wässrigen Lösung, um eine Öl-in-Wasser-Nanoemulsion zu bilden
- Gewinnen der erhaltenen Zusammensetzung der Nanoemulsion.

**15.** Zusammensetzung nach Anspruch 1 bis 13, zur Anwendung bei der Diagnose, vorteilhaft von Krebs-, entzündlichen, neurodegenerativen und/oder kardiovaskulären Erkrankungen.

## Claims

1. Oil-in-water nanoemulsion composition for MRI, comprising:

   - an aqueous phase, representing 70% to 90% by weight of the composition, advantageously 75% to 85% and more advantageously from 78% to 82%
   - a lipid phase comprising an oil, representing 9.5% to 29.5% by weight of the composition, advantageously 14% to 25% and more advantageously 17% to 21%,
   - a surfactant at the interface between the aqueous and lipid phases, the surfactant comprising at least one amphiphilic paramagnetic metal chelate, at least one amphiphilic targeting ligand and optionally an amphiphilic lipid;
   - the total content of surfactant by weight relative to the oil being between 4% and 10% and advantageously between 5% and 8%;
   - the total content of surfactant by weight relative to the composition being between 0.35% and 2.95% and advantageously between 0.5% and 2%;
   - the oil comprising at least 70%, advantageously at least 80%, advantageously at least 95% by weight and especially at least 97% of saturated C6-C18, advantageously C6-C14 and more advantageously C6-C10 fatty acids.

2. Composition according to Claim 1, **characterized in that** the amphiphilic targeting ligand comprised in the surfactant represents from 0.01% to 10% by weight of the total surfactant, advantageously 0.05% to 5% and especially 0.05% to 1%.

3. Composition according to Claim 1 or 2, **characterized in that** the surfactant represents 5% to 8% by weight of the oil.

4. Composition according to Claims 1 to 3, **characterized in that** the saturated fatty acids are in the form of saturated fatty acid triglycerides.

5. Composition according to Claims 1 to 3, **characterized in that** the oil comprises saturated fatty acids in the following proportions:

   - C6-C18 > 70%, advantageously > 80%, more advantageously > 95%, even more advantageously > 98%
   - or C6-C14 > 70%, advantageously > 80%, more advantageously > 95%, even more advantageously > 98%
   - or C8+C10 > 70%, advantageously > 80%, more advantageously > 95%, even more advantageously > 98%.

6. Composition according to any one of Claims 1 to 5, **characterized in that** the surfactant comprises:

   - 50% to 95% by weight of amphiphilic lipid,
   - 5% to 50% by weight of amphiphilic paramagnetic metal chelate, advantageously 5% to 30% by weight, and where appropriate
   - 0.05% to 5% by weight of an amphiphilic targeting ligand.

7. Composition according to any one of Claims 1 to 5, **characterized in that** the surfactant comprises 95% to 99.95% of amphiphilic paramagnetic metal chelate and 0.05% to 5% of an amphiphilic targeting ligand.

8. Composition according to any one of Claims 1 to 7, **characterized in that** the amphiphilic paramagnetic metal chelate is a macrocyclic chelate chosen from: DOTA, DO3A, HPDO3, BTDO3A, PCTA, DOTAM, DOTMA, DOTA-GA, AAZTA, HOPO, and also multimers thereof and derivatives thereof.

9. Composition according to any one of Claims 1 to 8, **characterized in that** the targeting ligand is a biovector chosen from: peptides (advantageously of less than 20 amino acids, more advantageously of 5 to 10 amino acids), pseudopeptides, peptidomimetics, amino acids, integrin targeting agents, glycoproteins, lectins, biotin, pteroic or aminopteroic derivatives, folic and antifolic acid derivatives, antibodies or antibody fragments, avidin, steroids, oligonucleotides, ribonucleic acid sequences, deoxyribonucleic acid sequences, hormones, proteins, which may be recombinant or muted, mono- or polysaccharides, compounds with a benzothiazole, benzofuran, styrylbenzoxazole/thiazole/imidazole/quinoline or styrylpyridine scaffold.

10. Composition according to any one of Claims 1 to 9, **characterized in that** the amphiphilic lipid is a phospholipid,

advantageously chosen from phosphatidylcholine, dioleoylphosphatidylcholine, dimyristoylphosphatidylcholine, dipalmitoylphosphatidylcholine, distearoylphosphatidylcholine, phosphatidylethanolamine, sphingomyelin, phosphatidylserine, phosphatidylinositol and lecithin.

**11.** Composition according to any one of Claims 1 to 10, **characterized in that** the amphiphilic targeting ligand is of the formula Bio - L - Lipo, in which:

- Bio is the biological recognition part located on the outer surface of the nanodroplets
- Lipo is a lipophilic group for inserting the lipophilic biovector into the surfactant layer
- L is a linking group connecting Bio and Lipo, advantageously chosen from:

    - nothing or a single bond, squarate, C1-6 alkylene, PEG, for example CH2-(CH2-O-CH2)k-CH2 with k = 1 to 10, $(CH_2)_3$-NH, NH-$(CH_2)_2$-NH, NH-$(CH_2)_3$-NH, $(CH_2)_n$, $(CH_2)_n$-CO-,-$(CH_2)_n$NH-CO- with n = 2 to 10, $(CH_2CH_2O)_q(CH_2)_r$-CO-, $(CH_2CH_2O)$q$(CH_2)_r$-NH-CO-with q = 1-10 and r=2-10, $(CH_2)_n$-CONH-, $(CH_2)_n$-CONH - PEG, $(CH_2)_n$-NH- HOOC-$CH_2$-O-$(CH_2)_2$-O-$(CH_2)_2$-O-$CH_2$-COOH; HOOC-$(CH_2)_2$-$CO_2$-$(CH_2)_2$-OCO-$(CH_2)_2$-COOH ; HOOC-CH(OH)-CH(OH)-COOH; HOOC-$(CH_2)_n$-COOH; $NH_2$-$(CH_2)_n$-$NH_2$, with n=0-20; $NH_2$-$(CH_2)_n$-$CO_2H$; $NH_2$-$CH_2$-$(CH_2$-O-$CH_2)_n$-$CO_2H$ with n=1 to 10,
    - P1-I-P2, which may be identical or different, P1 and P2 being chosen from O, S, NH, nothing, $CO_2$, NHCO, CONH, NHCONH, NHCSNH, $SO_2$NH-, $NHSO_2$-, squarate with 1 = alkyl, alkoxyalkyl, polyalkoxyalkyl (PEG), alkyl interrupted with one or more squarates or with one or more aryls, advantageously phenyls, alkenyl, alkynyl, alkyl interrupted with one or more groups chosen from -NH-, -O-, - CO-, -NH(CO)-, -(CO)NH-, -O(CO)-, or - (OC)O-.

**12.** Composition according to any one of Claims 1 to 11, **characterized in that** the surfactant also comprises an amphiphilic stealth agent, advantageously a PEG derivative, a ganglioside derivative, a polysaccharide.

**13.** Composition according to any one of Claims 1 to 12, **characterized in that** the paramagnetic metal of the amphiphilic paramagnetic metal chelate is chosen from: manganese, iron, cobalt, nickel, copper, molybdenum, ruthenium, cerium, praseodymium, neodymium, promethium, samarium, europium, gadolinium, terbium, dysprosium, holmium, erbium, thulium and ytterbium; advantageously from Gd(III), Mn(II), europium and dysprosium.

**14.** Process for preparing a composition according to any one of Claims 1 to 13, comprising the steps of:

- preparation of a lipid phase comprising

    ◦ optionally a first amphiphilic lipid surfactant
    ◦ an oil comprising at least 70% by weight, advantageously at least 80%, more advantageously at least 95% of C6-C18, advantageously C6-C14 and more advantageously C6-C10 saturated fatty acids
    ◦ an amphiphilic paramagnetic metal chelate
    ◦ an amphiphilic targeting ligand

- dispersion of the lipid phase in an aqueous solution so as to form an oil-in-water nanoemulsion
- recovery of the nanoemulsion composition obtained.

**15.** Composition according to Claims 1 to 13, for its use in diagnosis, advantageously of cancerous, inflammatory, neurodegenerative and/or cardiovascular diseases.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03062198 A
- US 6676963 B
- WO 2010018222 A
- US 20070148194 A
- WO 9533494 A
- WO 2006100305 A
- WO 0160416 A
- WO 03011115 A
- EP 499501 A
- EP 287465 A, Guerbet
- WO 0197850 A
- US 6372194 B
- WO 20019188 A
- WO 0177145 A
- WO 0226776 A
- WO 02081497 A
- WO 9940947 A
- WO 02062810 A
- WO 0240060 A
- US 6524554 B
- WO 02094873 A
- US 6489333 B
- US 6511648 B
- US 20020106325 A
- WO 0197861 A
- WO 0198294 A
- WO 0110450 A
- US 6261535 B
- US 5707605 A
- WO 0228441 A
- WO 02056670 A
- US 6410695 B
- US 6391280 B
- WO 9954317 A
- US 6491893 B
- US 20020128553 A
- WO 02054088 A
- WO 0232292 A
- WO 0238546 A
- WO 03006059 A
- US 6534038 B
- WO 0177102 A
- EP 1121377 A
- WO 0244156 A
- WO 02059110 A
- WO 9400489 A
- WO 9405269 A
- WO 02066512 A
- EP 0425212 A
- US 6537520 B
- WO 2003014145 A
- WO 0320701 A
- WO 0311115 A
- WO 02085903 A
- US 2007098631 A
- WO 2009114776 A
- WO 2007042506 A
- US 6010682 A
- WO 2010066815 A
- WO 2004112839 A


### Littérature non-brevet citée dans la description


- **H. MAEDA ; J. WU ; T. SAWA ; Y. MATSUMURA ; K. HORI.** *Tumor vascular permeability and the EPR effect in macromolecular therapeutics : A review, J. Control. Release,* 2000, vol. 65, 271-284
- *Mini Reviews in Medicinal Chemistry,* 2003, vol. 3 (8
- *Tetrahedron Letters,* 1980, vol. 22 (18), 1711-1714
- **MÜLLER et al.** *Eur. J. Org. Chem,* 2002, 3966-3973
- *Pharmacological Reviews,* vol. 52 (2), 179
- **W.KRAUSE, SPRINGER.** *Bioorganic & Medicinal Chemistry,* 2003, vol. 11, 1319-1341
- *Nuclear Medicine Communications,* 1999, vol. 20
- *Current Chemistry,* vol. 222, 260-274
- *J Med. Chem.,* 2003, vol. 46, 4790-4798
- *Bioorg. Med. Chem. Letters,* 2004, vol. 14, 4515-4518
- *Bioorg. Med. Chem. Letters,* 2005, vol. 15, 1647-1650